# EUROPEAN PATENT SPECIFICATION

(11) **EP 0 284 603 B1**
(45) Date of publication and mention of the grant of the patent: **07.01.1998**
(21) Application number: 86902449.7
(22) Date of filing: 14.04.1986
(51) Int. Cl.: C12N 15/00, C12N 15/80

(54) **USE OF PROMOTERS OF FILAMENTOUS FUNGI**
VERWENDUNG VON PROMOTOREN VON FILAMENTÖSEN PILZEN
UTILISATION D'ACTIVEURS DE CHAMPIGNONS FILAMENTEUX

(30) Priority: 15.04.1985 CA 479135; 20.12.1985 US 811404
(43) Date of publication of application: 05.10.1988
(60) Divisional application: 96116202.1
(73) Proprietor: GIST-BROCADES N.V., NL-2600 MA Delft (NL)
(72) Inventor: GWYNNE, David, Ivor 34 Monterrey Avenue, Ontario (CA); BUXTON, Francis, Paul 727 Indian Road, Ontario (CA); PICKETT, Mark, Hudson, Ontario (CA); DAVIES, Roger, Wayne R. R. 1, Ontario (CA); SCAZZOCCHIO, Claudio 6 parc de Bures, F-91440 Bures sur Yvette (FR)
(74) Representative: Matulewicz, Emil Rudolf Antonius, Dr.
(86) International application number: GB8600209
(87) International publication number: WO8606097

(56) References cited:
- EP-A- 0 099 226
- EP-A- 0 126 206
- EP-A- 0 127 304
- EP-A- 0 136 907
- EP-A- 0 173 378
- EP-A- 0 181 213
- PNAS USA, Vol. 80, pp. 7576-7580 (1983)
- Third European Congress on Biotechnology, München 10-14 Sept. 1984, Vol. III, Verlag Chemie, Weinheim, pages 305-310
- CHEMICAL ABSTRACTS, vol. 98, no. 25, 20 June 1983 Columbus, Ohio, US; D.J. BALLANDE et al.: "Transformation of Aspergillus nidulans by the orotidine 5'-phosphate decarboxylase gene of Neurospora crassa", see page 150, abstract no. 210814t & BIOCHEM. BIOPHYS. RES. COMMUN. 1983, 112(1), 284-9
- CHEMICAL ABSTRACTS, vol. 103, 29 July 1985 Columbus, Ohio, US; C.H. DOY et al.: "Genomic clones of Aspergillus nidulans containing alcA, the structural gene for alcohol dehydrogenase and alcR, a regulatory gene for ethanol metabolism" see page 153, abstract no. 17752q & DNA 1985, 4(2), 105-14
- CHEMICAL ABSTRACTS, vol. 101, no. 17, 22 October 1984 Columbus, Ohio, US; E. BOEL et al.: "Two different types of intervening sequences in the glucoamylase gene from Aspergillus niger", see page 158, abstract no. 145009y & EMBO J. 1984, 3(7), 1581-5
- GENE, Vol. 33, 1985, Elsevier Science Publ., R. A. LOCKINGTON et al., "Cloning and Characterization of the Ethanol Utilization Regulon in Aspergillus Nidulans", pp. 137-149.
- CHEMICAL ABSTRACTS, Vol. 104, 1986 ,Columbus, Ohio, US; C.A.M.J.J. VAN DEN HONDEL et al., "Development of a System for Analysis of Regulation Signals in Aspergillus", page 167, Abstract No. 103393a & SYMP. MOL. CELL. BIOL., New Ser. 1985, 34 (Mol. Genet. Filamentous Fungi), 29-38.
- GENE, Vol. 37, 1985, Elsevier Science Publishers, F. P. BUXTON et al., "Transformation of Aspergillus Niger Using the argB Gene of Aspergillus Nidulans", pp. 207-214, See Summary.
- CHEMICAL ABSTRACTS, Vol. 98, No. 19, 9 May 1983, Columbus, Ohio, US; J. A. PATEMAN et al., " Regulation of Alcohol Dehydrogenase (ADH) and Aldehyde Dehydrogenase (AldDH) in Aspergillus Nidulans", page 247, Abstract No. 157629h & PROC. R. SOC. LONDON, (Ser) B 1983, 217(1208), 243-64.
- NATURE, Vol. 308, No. 5960, April 1984, Reading, Berks, GB; S. J. ROTHSTEIN et al., "Secretion of a Wheat Khi-Amylase Expressed in Yeast", Pages 662-665, see whole document.
- GENE, Vol. 17, 1982, Elsevier Biomedical Press, D. J. WHITTLE et al., "Molecular Cloning of a Cellulomonas Fimi Cellulase Gene in Escherichia Coli", pp. 139-145, see summary.
- GENE, Vol. 12, 1980, Elsevier Biomedical Press, K. TALMADGE et al., "Construction of Plasmid Vectors with Unique PstI Cloning Sites in a Signal Sequence Coding Region", pp. 235-241, see summary.
- CHEMICAL ABSTRACTS, Vol. 100, No. 5, 30 January 1984, Columbus, Ohio, US; J. A. PATEMAN et al., "Genes for Alcohol Utilization in the Lower Eukaryote Aspergillus Nidulans", page 108, Abstract No. 30390c & MANIPULATION EXPRESSED GENES EUKARYOTES PRO. INT. CONF., INT. CONGR. BIOCHEM., 12th 1982 (Publ. 1983) 171-8.

## Description

This invention relates to expression and expression followed by secretion of proteins from filamentous fungi.

### BACKGROUND OF THE INVENTION

One goal of recombinant DNA technology is the insertion of DNA segments which encode commercially or scientifically valuable proteins into a host cell which is readily and economically available. Genes selected for insertion are normally those which encode proteins produced in only limited amounts by their natural hosts or those which are indigenous to hosts too costly to maintain. Transfer of the genetic information in a controlled manner to a host which is capable of producing the protein in either greater yield or more economically in a similar yield provides a more desirable vehicle for protein production.

Genes encoding proteins contain promoter regions of DNA which are essentially attached to the 5' terminus of the protein coding region. Tne promoter regions contain the binding site for RNA polymerase II. RNA polymerase II effectively catalyses the assembly of the messenger RNA complementary to the appropriate DNA strand of the coding region. In most promoter regions, a nucleotide base sequence related to the sequence known generally as a "TATA box" is present and is generally disposed some distance upstream from the start of the coding region and is required for accurate initiation of transcription. Other features important or essential to the proper functioning and control of the coding region are also contained in the promoter region, upstream of the start of the coding region.

Filamentous fungi, particularly the filamentous ascomycetes such as Aspergillus, e.g. Aspergillus niger, represent a class of micro-organisms suitable as recipients of foreign genes coding for valuable proteins. Aspergillus niger and related species are currently used widely in the industrial production of enzymes e.g. for use in the food industry. Their use is based on the secretory capacity of the microorganism. Because they are well characterized and because of their wide use and acceptance, there is both industrial and scientific incentive to provide genetically modified and enhanced cells of A. niger and related species including A. nidulans, in order to obtain useful proteins.

Expression and secretion of foreign proteins from filamentous fungi has not yet been achieved. It is by no means clear that the strategies which have been successful in yeast would be successful in filamentous fungi such as Aspergillus. Evidence has shown that yeast is an unsuitable system for the expression of filamentous fungal genes (Pentilla et al Molec. Gen. Genet. (1984) 194:494-499) and that yeast genes do not express in filamentous fungi. Genetic engineering techniques have only recently been developed for Aspergillus nidulans and Aspergillus niger. These techniques involve the incorporation of exogenously added genes into the Aspergillus genome in a form in which they are able to be expressed.

To date no foreign proteins have been expressed in and secreted from filamentous fungi using these techniques. This has been due to a lack of suitable expression vectors and their constituent components. These components include Aspergillus promoter sequences described above, the region encoding the desired product and the associated sequences which may be added to direct the desired product to the extracellular medium.

As noted, expression of the foreign gene by the host cell requires the presence of a promoter region situated upstream of the region coding for the protein. This promoter region is active in controlling transcription of the coding region with which it is associated, into messenger RNA which is ultimately translated into the desired protein product. Proteins so produced may be categorized into two classes on the basis of their destiny with respect to the host.

A first class of proteins is retained intracellularly. Extraction of the desired protein, when intracellular, requires that the genetically engineered host be broken open or lysed in order to free the product for eventual purification. Intracellular production has several advantages. The protein product can be concentrated i.e. pelleted with the cellular mass, and if the product is labile under extracellular conditions or structurally unable to be secreted, this is a desired method of production and purification.

A second class of proteins are those which are secreted from the cell. In this case, purification is effected on the extracellular medium rather than on the cell itself. The product can be extracted using methods such as affinity chromatography and continuous flow fermentation is possible. Also, certain products are more stable extracellularly and are benefited by extracellular purification. Experimental evidence has shown that secretion of proteins in eukaryotes is almost always dictated by a secretion signal peptide (hereafter called signal peptide) which is usually located at the amino terminus of the protein. Signal peptides have characteristic distributions as described by G. Von Heijne in Eur. J. Biochem 17-21 (1983) and are recognizable by those skilled in the art. The signal peptide, when recognized by the cell, directs the protein into the cell's secretory pathway. During secretion, the signal peptide is cleaved off making the protein available for harvesting in its mature form from the extracellular medium.

Both classes of protein, intracellular and extracellular, are encoded by genes which contain a promoter region coupled to a coding region. Genes encoding extracellularly directed proteins differ from those encoding intracellular proteins in that, in genes encoding extracellular proteins, the portion of the coding region nearest to the promoter (which is the first part to be transcribed by RNA polymerase) encodes a signal peptide. The nucleotide sequence encoding the signal peptide, hereafter denoted the signal peptide coding region or the signal sequence, is operationally part of the coding region per se.

### SUMMARY OF THE INVENTION

A system has now been developed by which filamentous fungi may be tranformed to express a desired protein. With this system, transformation can result in a filamentous fungus which is capable not only of expressing the protein but of secreting that protein as well, regardless of whether or not the protein is a naturally secreted one. In addition, the level at which the protein is expressed can be controlled according to certain aspects of the invention. It will be appreciated by those skilled in the art that the system provided permits filamentous fungi to function as valuable source of proteins and provides an alternative which in many applications is superior to bacterial and yeast systems.

Thus, in a general aspect, the invention provides a filamentous fungus transformation system by which the genetic constitution of these fungus cells may be modified so as to alter either the nature of the amount of the proteins expressed by these cells. More specific aspects of the invention are defined below.

According to the present invention there is provided a recombinant DNA construct comprising a regulatable promoter region of a filamentous fungus gene operably linked to a DNA fragment encoding the polypeptide, wherein:
(a) said DNA fragment is foreign to the promoter region; and
(b) said regulatable promoter region is inducible by ethanol said ethanol inducibility being mediated by the alcR gene product.

There is yet further provided a method for producing a polypeptide which comprises:
(i) culturing a filamentous fungus which has been transformed by a recombinant DNA construct in which a regulatable promoter region of a filamentous fungus gene is operably linked to a DNA fragment encoding the polypeptide, wherein:
   (a) said DNA fragment is foreign to the promoter region; and
   (b) said regulatable promoter region is inducible by ethanol said ethanol inducibility being mediated by the alcR gene product;
(ii) under conditions in which regulated expression of the polypeptide occurs; and
(iii) recovering said polypeptide.

There is further provided a filamentous fungus which is transformed with a recombinant DNA construct of the invention.

In the present invention, from one aspect, a promoter DNA region associated with a coding region in filamentous fungi such as A. niger, A. nidulans or a related species is identified and isolated, appropriately joined in a functional relationship with a second, different DNA coding region, outside the cell, and then re-introduced into a host filamentous fungus using an appropriate vector. Transformed host cells express the protein of the second coding region, under the control of the introduced promoter region. The second coding region may be one which is foreign to the host species, in which case the host will express and in some cases secrete a protein not naturally expressed by the given host.

The present invention provides the ability to introduce foreign coding regions into filamentous fungi along with promoters to arrange for the host fungi to express different proteins. It also provides the ability to regulate transcription of the individual genes which occur naturally therein or foreign genes introduced therein, via the promoter region which has been introduced into the host along with the gene. For example, the promoter region naturally associated with the alcohol dehydrogenase I (alcA) gene and the aldehyde dehydrogenase (aldA) gene of A. nidulans are regulatable by means of ethanol, threonine, or other inducing substances in the extracellular medium. This effect is dependent on the integrity of a gene known as alcR. When the alcA or aldA promoter region is associated with a foreign protein coding region in Aspergillus or the like, in accordance with the present invention, similar regulation of the expression of the different genes by ethanol or other inducers can be achieved.

In another aspect, the present invention provides a genetic vector capable of introducing the segment carrying the above mentioned regulatable promoter and signal peptide coding region with integral protein coding region into the genome of a filamentous fungus host. The protein coding region can be either native to or foreign to the host filamentous fungus.

The present invention thus also provides a novel construct comprising a DNA sequence the promoter region mentioned above in cells of filamentous fungi, and a coding region chemically bound to said DNA sequence in operative association therewith, said coding region being capable of expression in a filamentous fungus host under influence of said DNA sequence.

The present invention further provides a process of genetically modifying a filamentous fungus host cell which comprises introducing into the host cell, by means of a suitable vector, a coding region capable of expression in the transformed Aspergillus host cell and a promoter region as described above active in the transformed Aspergillus host cell, the coding region which is foreign to the promoter region and the promoter being chemically bound together and in operative association with one another.

This process also encompasses the introduction of multiple copies of the selected construct into the host to provide for enhanced levels of gene expression. If necessary or desirable, introduction of multiple construct copies is accompanied by introduction of multiple copies of genes encoding products having a regulatory effect on the construct.

The present invention also comprises filamentous fungal cells transformed by the constructs of the invention.

### DESCRIPTION OF THE PREFERRED EMBODIMENTS

Preferred hosts according to the invention are the filamentous fungi of the ascomycete class, most preferably Aspergillus sp. including A. niger, A. nidulans and the like.

In the preferred form of the invention the promoter region associated with either the Aspergillus niger glucoamylase gene or the promoter region associated with either the alcohol dehydrogenase I gene or aldehyde dehydrogenase genes of Aspergillus nidulans is used in preparing an appropriate vector plasmid.

Either or all of these promoter regions is regulatable in the host cell by the addition of the appropriate inducer substance. In alcA and aldA, this induction is mediated by the protein product of a third gene, alcR which is controlled via the promoter. Evidence indicates that the availability of alcR product can limit the promoting function of the alcA and aldA promoters when multiple copies of a construct containing the alcA promoter or the aldA promoter are introduced into a host without corresponding introduction of multiple copies of the alcR gene. In such a case, the amount of alcR product which the host can produce may be insufficient to meet the demands of the several promoters requiring induction by the alcR product. Thus, transformation of filamentous fungal hosts by multiple copies of constructs containing the alcA or aldA promoter is accompanied by introduction of multiple copies of the alcR gene, according to a preferred embodiment of the present invention. In other instances, transcription can be repressed, for example by utilizing high levels of glucose, (and some other carbon sources) in the medium to be used for growth of the host. The expression of the product encoded by the coding region and controlled by the promoter is then delayed until after the end of the cell growth phase, when all of the glucose has been consumed and the gene is derepressed. The inducer may be added at this point to enhance the activity of the promoter.

The destination of the protein product of the coding region which has been selected to be expressed under the control of the promoter described above is determined by the nucleotide sequence of that coding region. As mentioned, if the protein product is naturally directed to the extracellular environment, it will inherently contain a secretion signal peptide coding region. Protein products which are normally intracellularly located lack this signal peptide.

Thus, for the purposes of the present disclosure it is to be understood that a "coding region" encodes a protein which is either retained intracellularly or is secreted. (This "coding region" is sometimes referred to in the art as a structural gene i.e. that portion of a gene which encodes a protein.) Where the protein is retained within the cell that produces it, the coding region will usually lack a signal peptide coding region. Secretion of the protein encoded within the coding region can be a natural consequence of cell metabolism in which case the coding region inherently contains a signal peptide coding region linked naturally in translation reading frame with that segment of the coding region which encodes the secreted protein. In this case, insertion of a signal peptide coding region is not required. In the alternative, the coding region may be manipulated to introduce a signal peptide coding region which is foreign to that portion of the coding region which encodes the secreted protein. This foreign signal peptide coding region may be required where the coding region does not naturally contain a signal peptide coding region or it may simply replace the natural signal peptide coding region in order to obtain enhanced secretion of the desired protein with which the natural signal peptide is normally associated.

In accordance with another preferred aspect of the invention, therefore, a signal peptide coding region is provided, if required i.e. when the coding region which has been selected to be expressed under the control of the promoter described above does not itself contain a signal peptide coding region. The signal peptide coding region used is preferably either one which is associated with the Aspergillus niger glucoamylase gene or a synthetic signal peptide coding region which is made in vitro and used in the preparation of an appropriate vector plasmid. Most preferably, these signal peptide coding regions are modified at one or both termini to permit ligation thereof with other components of a vector. This ligation is effected in such a way that the signal peptide coding region is interposed between the promoter region and the protein encoding segment of the coding region such that the signal peptide coding region is in frame with that segment of the coding region which encodes the mature, functional protein.

### BRIEF REFERENCE TO THE DRAWINGS

Figure 1A is an illustration of the base sequence of the DNA constituting the coding region and promoter region of the alcohol dehydrogenase I (alcA) gene of Aspergillus nidulans.
Figure 1B is an illustration of the base sequence of DNA constituting the coding region and promoter region of the aldehyde dehydrogenase (aldA) gene of Aspergillus nidulans.
Figure 2 is a diagrammatic illustration of a process of constructing plasmid pDG6 useful in transforming a filamentous fungal cell;
Figure 3 is a linear representation of a portion of the plasmid pDG6 of Fig.2;
Figure 4 is a diagrammatic illustration of the plasmid maps of pGLl and pGL2;
Figure 5 is an illustration of a selection of synthetic linker sequences for insertion into plasmid pGL2;
Figure 6 is an illustration of the nucleotide sequence of a fragment of pGL2;
Figure 7 is an illustration of plasmid map pGL2B and pGL2BIFN;
Figure 8 is an illustration of the nucleotide sequence of a fragment of pGL2BIFN;
Figure 9 illustrates plasmid pALCAIS and a method for its preparation;
Figure 10 illustrates the plasmid map of pALCAlSIFN and a method for its preparation;
Figure 11 represents the nucleotide sequence of a fragment of pALCAlSIFN;
Figure 12 illustrates the plasmid map of pGL2CENDO;
Figure 13 represents the nucleotide sequence of a fragment of pGL2CENDO;
Figure 14 represents a plasmid map of pALCAlSENDO;
Figure 15 represents the nucleotide sequence of a fragment of pALCAlSENDO;
Figure 16 illustrates plasmid pALCAlAMY and a method for its preparation; and
Figure 17 represents the nucleotiae sequence of a segment of pALCAlAMY shown in Figure 16.

### DETAILED DESCRIPTION OF PREFERRED EMBODIMENTS

In the present invention, an appropriate promoter region of a functioning gene in A. niger or A. nidulans or the like is identified. Procedures for identifying each of the genes containing the desired promoter regions are similar and for that reason, the manner of locating and identifying the alcA gene and promoter therein is outlined. For this purpose, cells of the chosen species are induced to express the selected protein e.g. alcA, and from these cells is isolated the messenger RNA. One portion thereof, as yet unidentified codes for alcA. Complementary DNA for the fragments is prepared from the mRNA fragments and cloned into a vector. Messenger RNA isolated from induced A. nidulans is size fractionated to enrich for alcA sequences, end labelled and hybridized to the cDNA clones made from the alcA⁺ strain. That clone containing the cDNA which hybridizes to alcA⁺ mRNA contains the DNA copy of the alcA mRNA. This piece is hybridized to a total DNA gene bank from the chosen Aspergillus species, to isolate the selected coding region e.g. alcA and its flanking regions. The aldA coding region was isolated using analogous procedures.

The coding region starts at its 5' end, with a codon ATG coding for methionine, in common with other coding regions and proteins. Where the amino acid sequence of the expressed protein is known, the DNA sequence of the coding region is readily recognizable. Immediately "upstream" of the ATG codon is the leader portion of the messenger RNA preceded by the promoter region.

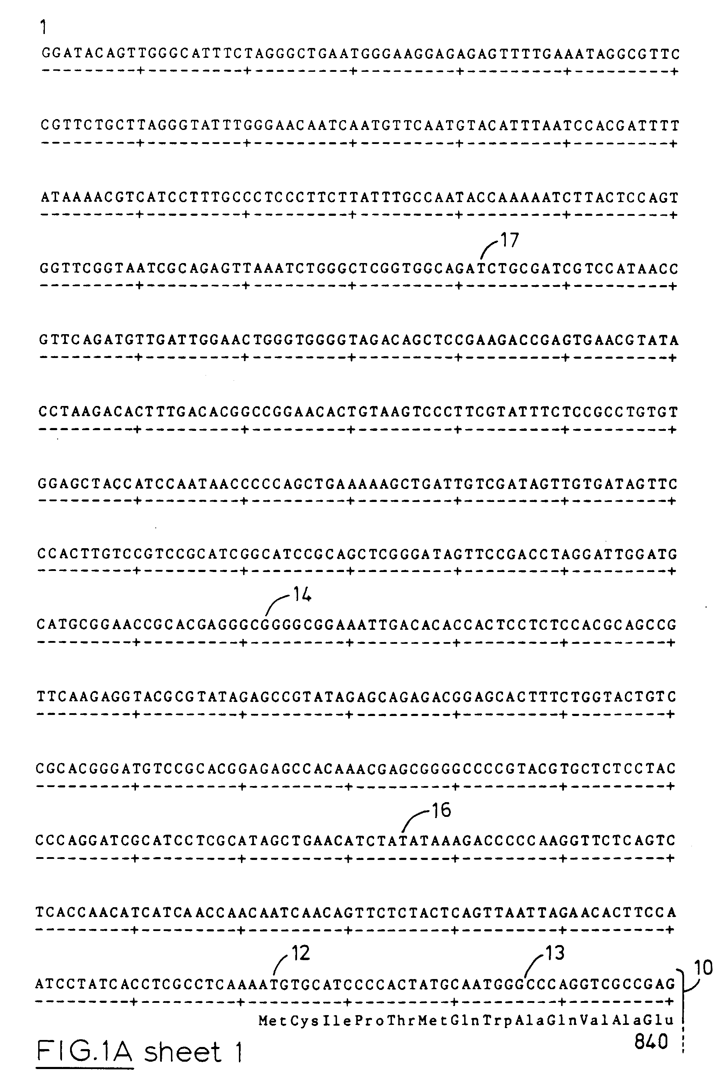

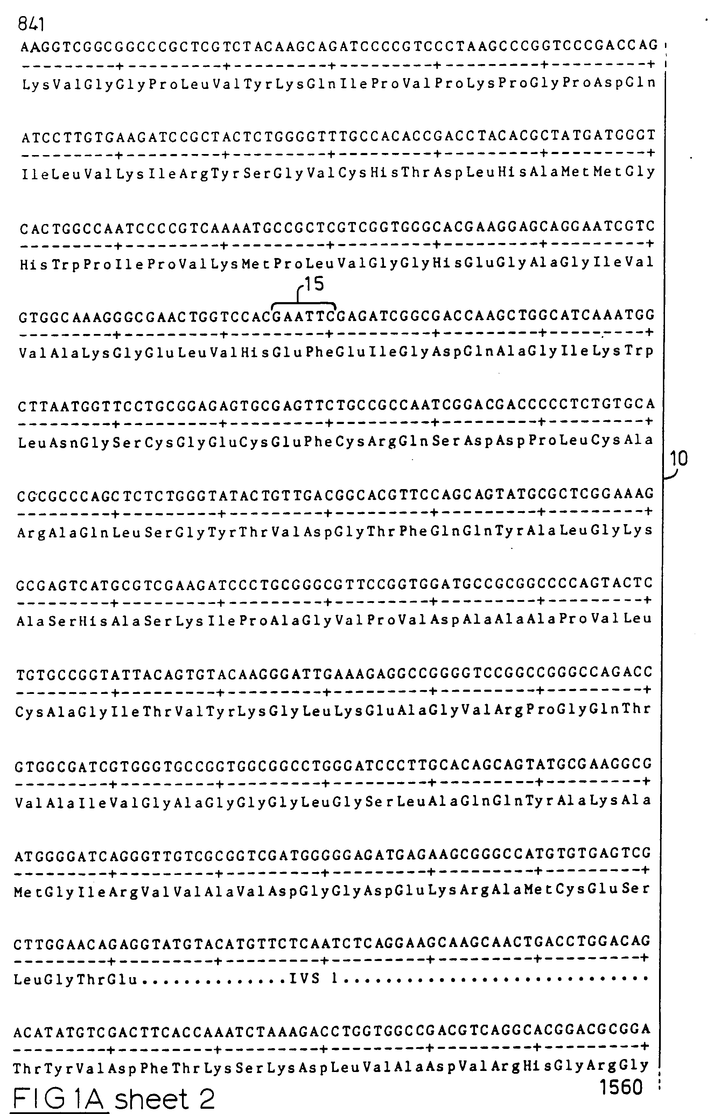

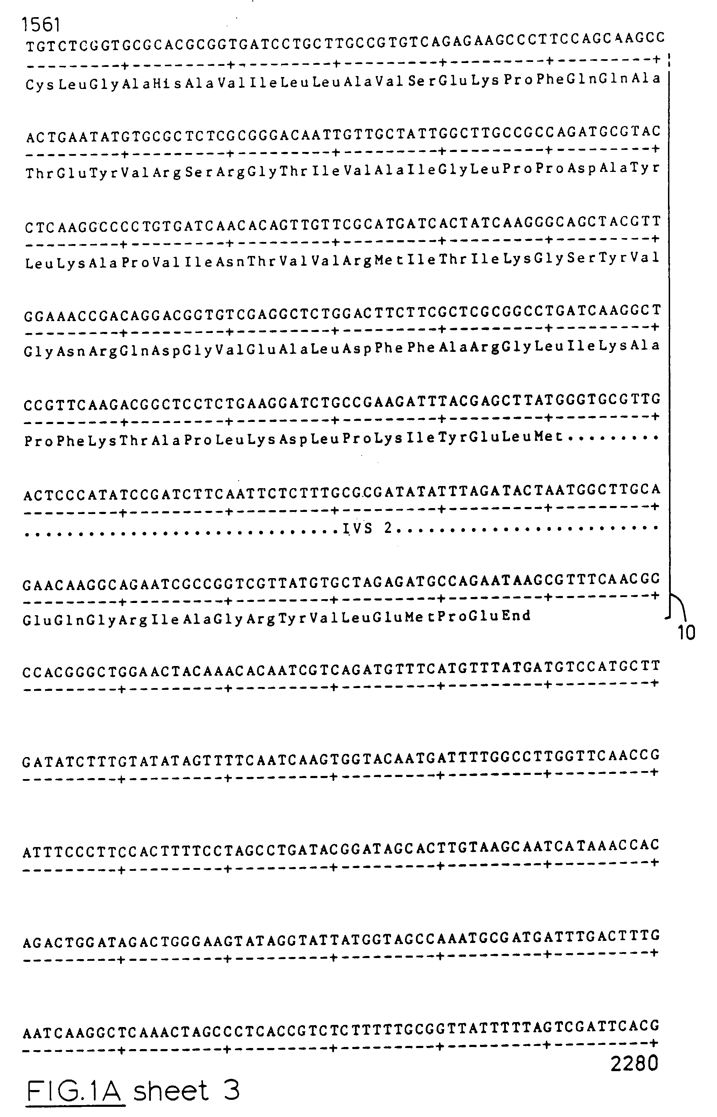

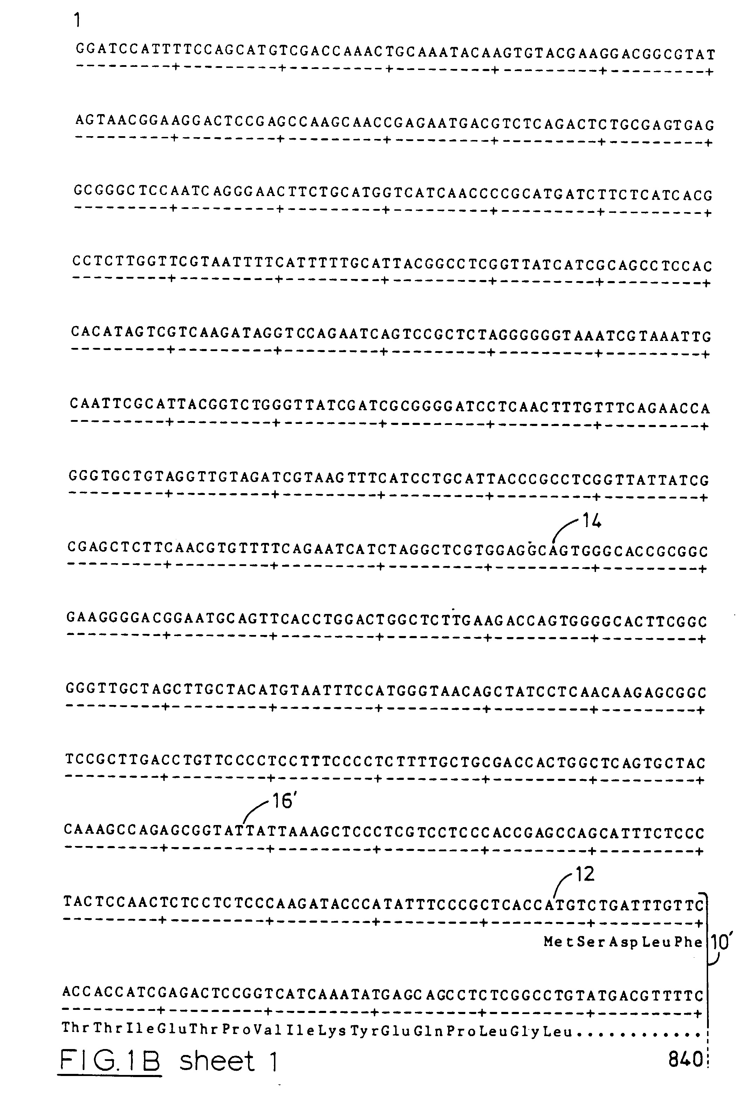

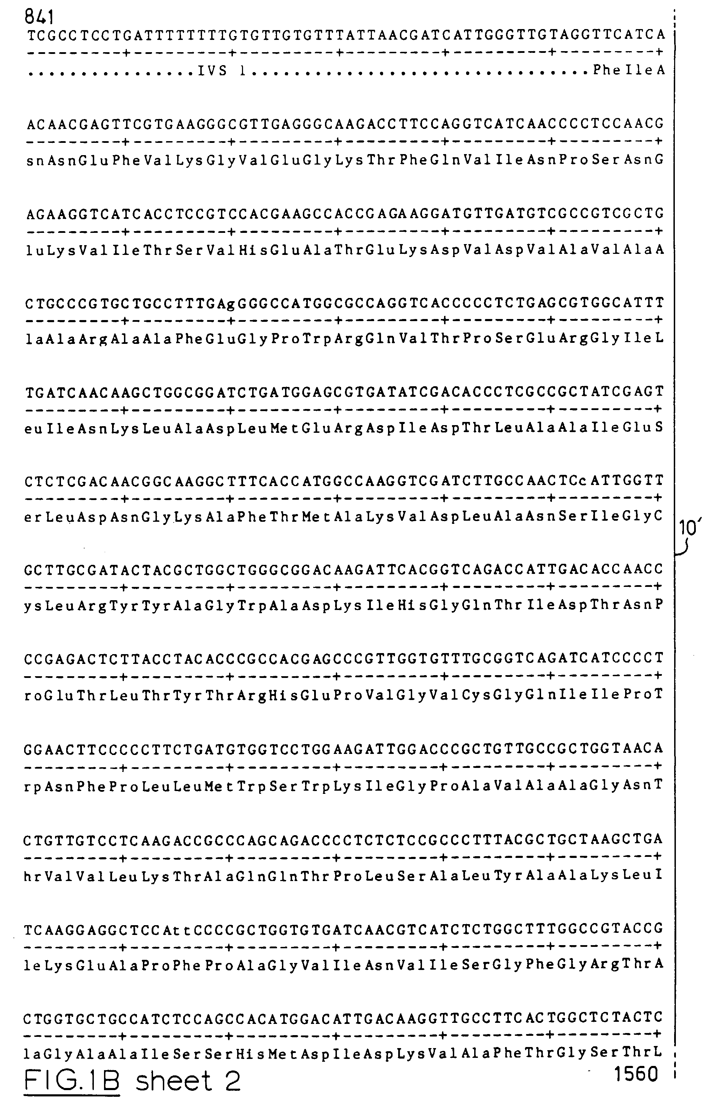

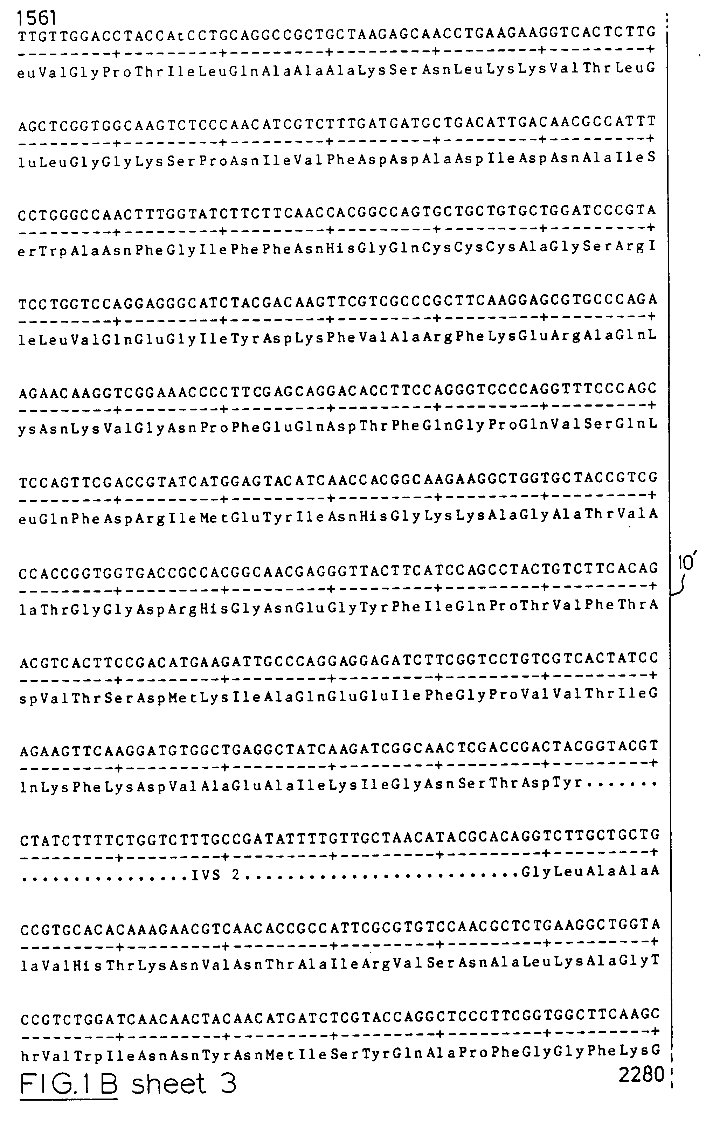

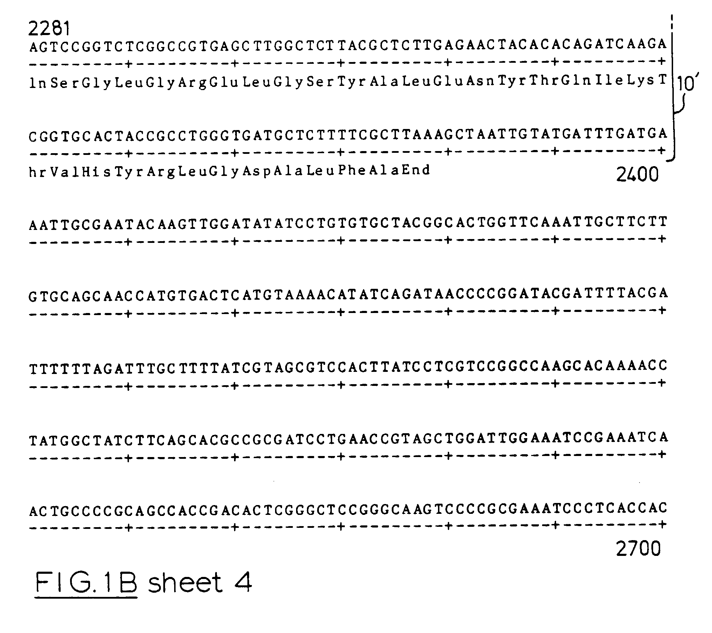

With reference to Figs. 1A and 1B, these show portions of the total DNA sequence from A. nidulans, with conventional base notations. The portion shown in Figure 1A contains the promoter region and the coding region of the alcA gene which encodes the enzyme alcohol dehydrogenase I. The portion shown in the Figure 1B contains the promoter region and the region encoding the enzyme aldehyde dehydrogenase i.e. aldA. (In both cases, the term "IVS" represents intervening sequences.) The amino acid sequences of these two enzymes is known in other species. From these, the regions 10 and 10' are recognisable as the coding regions. Each coding region starts at its 5' ("upstream") end with methionine codon ATG at 12. The appropriate amino acid sequences encoded by the protein coding region are entered below the respective rows on Figs. 1A and 1B, in conventional abbreviations. Immediately upstream of codon 12 is the region coding for the messenger RNA leader and the promoter region, the length of which, in order to contain all the essential structural features enabling it to function as a promoter, now needs to be determined or at least estimated. Each of Figures 1A and 1B shows a sequence of about 800 bases in each case, upstream from the ATG codon 12.

It is predictable from analogy with other known promoters that all the functional essentials are likely to be contained within a sequence of about 1000 bases in length, probably within the 800 base sequence illustrated, and most likely within the first 200 - 300 base sequence, i.e. back to about position 14 on Figs. 1A and 1B. An essential function of a promoter region is to provide a site for accurate initiation of transcription, which is known to be a TATA box sequence. Such a sequence is found at 16 on the alcA promoter sequence of Fig. 1A, and at 16' on the aldA promoter sequence of Fig. 1B. Another function of a promoter region is to provide an appropriate DNA sequence active in regulation of the gene transcription, e.g. a binding site for a regulatory molecule which enhances gene transcription, or for rendering the gene active or inactive. Such regulator regions are within the promoter region illustrated in Figs. 1A and 1B for the alcA and aldA genes, respectively.

The precise upstream 5' terminus of the DNA sequence used herein as a promoter region is not critical, provided that it includes the essential functional sequences as described herein. Excess DNA sequences upstream of the 5' terminus are unnecessary, but unlikely to be harmful in the present invention.

Having determined the extent of the sequence containing all the essential functional features to constitute a promoter region of the given gene, by techniques described herein, the next step is to cut the DNA chain at a convenient location downstream of the promoter region terminus and to remove the protein coding region, to leave basically a sequence comprising the promoter region and sometimes part of the region coding for the messenger RNA leader. For this purpose, appropriately positioned restriction sites are to be located, and then the DNA treated with the appropriate restriction enzymes to effect scission. Restriction sites are recognizable from the alcA sequence illustrated in Figure 1A. For the upstream cutting, a site is chosen sufficiently far upstream to include in the retained portion all of the essential functional sites for the promoter region. As regards the downstream scission, no restriction site presents itself exactly at the ATG codon 12 in the case of alcA. The closest downstream restriction site thereto is the sequence GGGCCC at 13, at which the chain can be cut with restriction enzyme Apa I. If desired, after such scission, the remaining nucleotides from location 13 to location 12 can be removed, in stepwise fashion, using an exonuclease. With knowledge of the number of such nucleotides to be removed, the exonuclease action can be appropriately stopped when the location 12 is passed. By locating a similar restriction site downstream of the methionine codon 12 of the aldA coding region shown in Fig. 1B, this promoter region is similarly excised for subsequent use. In many cases, residual nucleotides on the 5' terminus of the promoter region are not harmful to and do not significantly interfere with the functioning of the promoter region, so long as the reading frame of the base triplets is maintained.

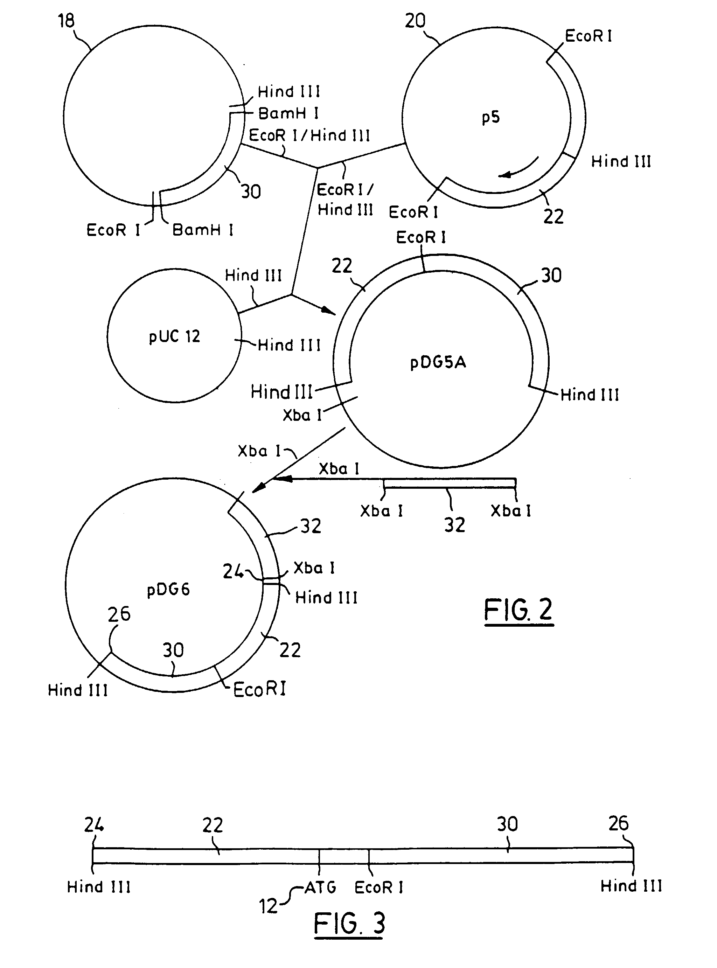

Fig. 2 of the accompanying drawings illustrates diagrammatically the steps in a process of preparing plasmid pDG6 which can be used to create Aspergillus transformants according to the present invention. On Fig. 2, 18 is a recombinant plasmid containing the endogluconase (cellulase) coding region 30 from the bacterium Cellulomonas fimi, namely a BamHI endoglucanase fragment from C. fimi in known vector M13MP8. It contains relevant restriction sites for EcoRI, Hind III and BamHI as shown as well as others not shown and not of consequence in the present process. Item 20 is a recombinant plasmid designated p5, constructed from known E. coli plasmid pBR322 and containing an EcoRI fragment of A. nidulans containing the alcA promoter region prepared as described above, along with a small portion of the alcA coding region, including the start codon ATG. It has restriction sites as illustrated, as well as other restriction sites not used in the present process and so not illustrated. Plasmid p5 contains a DNA sequence 22, from site EcoRI (3') to site Hind III (5'), which is in fact a part of the sequence illustrated on Fig. 1A, upper row, from position 15 (the sequence GAATTC thereat constituting an EcoRI restriction site) to position 17. Sequence 22 in plasmid 20 is approximately 2 kb in length.

The plasmids 18 and 20 are next cut with restriction enzymes EcoRI and Hind III, so as to excise the alc A promoter region and the endoglucanase coding region 30 which are ligated to Hind III-cut plasmid pUC12, to form a novel construct pDG5A containing these sequences on pUC12, as shown in Fig. 2. Plasmid pUC12 is a known, commercially available E. coli plasmid, which replicates efficiently in E. coli, so that abundant copies of pDG5A can be made if desired. Novel construct pDG5A is isolated from the other products of the construct preparation. Next, construct pDG5A is provided with a selectable marker so that subsequently obtained transformants of Aspergillus into which the construct has successfully entered can be selected and isolated. In the case of Arg B⁻ Aspergillus hosts, one can suitably use an Arg B gene from A. nidulans for this purpose. The Arg B gene codes for the enzyme ornithine transcarbamylase, and strains containing this gene are readily selectable and isolatable from Arg B⁻ strains by standard plating out and cultivation techniques. Arg B⁻ strains will not grow on a medium lacking arginine.

To incorporate a selectable marker, in this embodiment of the invention as illustrated in Fig. 2, construct pDG5A may be ligated with the Xba I fragment 32 of plasmid pDG3 ATCC 53006 (see U.S. patent application serial number 06/678,578 Buxton et al, filed December 5, 1984) which contains the Arg B⁺ gene from A. nidulans using Xba I, to form novel construct pDG6, which contains the endoglucanase coding region, the alcA promoter sequence and the Arg B gene. Plasmid pDG6 is then used in transformation, to prepare novel Aspergillus mutant strains containing an endoglucanase coding region under the control of alcA promoter, as described in more detail in Example 1.

Fig. 3 shows in linear form the diagrammatic sequence of the functional portion of construct pDG6, from the Hind III site 24 to the Hind III site 26. It contains the alcA promoter region 22, the ATG codon 12 and a small residual portion of the alcA coding region downstream of the ATG codon as shown in Fig. 1, followed by the cellulase coding region 30 derived from plasmid 18.

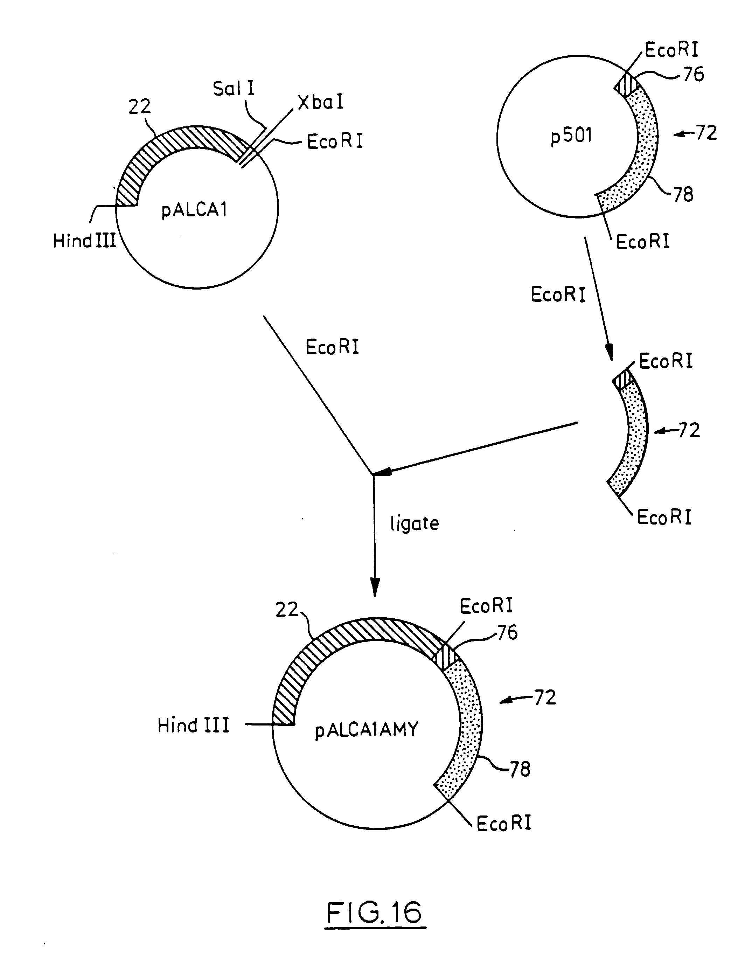

Plasmid pDG6 is but one example of a vector which contains a filamentous fungal promoter linked to a protein coding region foreign to the fungus. In another vector exemplified herein with reference to Figure 16 and referred to herein as pALCAlAMY, the filamentous fungal promoter of the alcA gene is coupled with the naturally occuring sequence coding for the α-amylase enzyme, a product which is foreign to the transformed fungal host. The protein products of vectors pDG6 and pALCAlAMY are expressed by the respective transformed hosts in both cases. Further, because the α-amylase coding region naturally contains a signal peptide coding region, this product can be secreted by the transformed host, using the secretory machinery of the host, despite its foreign relationship with the host.

Identifying and isolating the promoter regions of filamentous fungi thus allows one to manipulate the host by transformation with vectors containing these promoter regions coupled with a desired coding region.

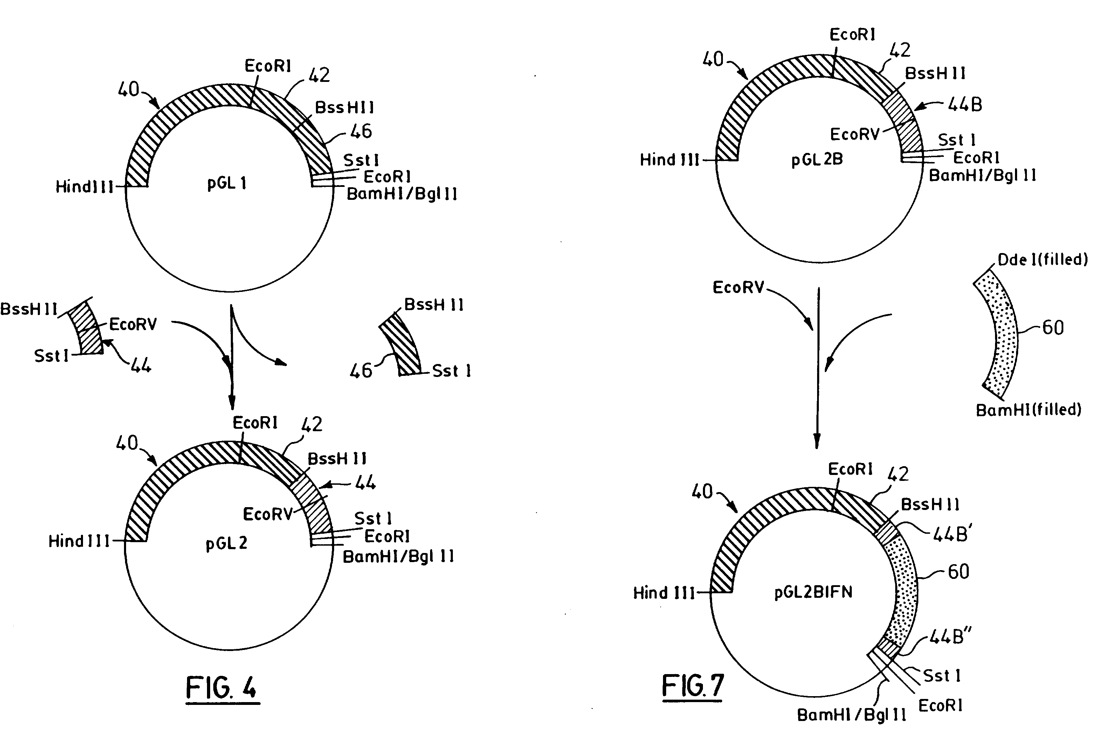

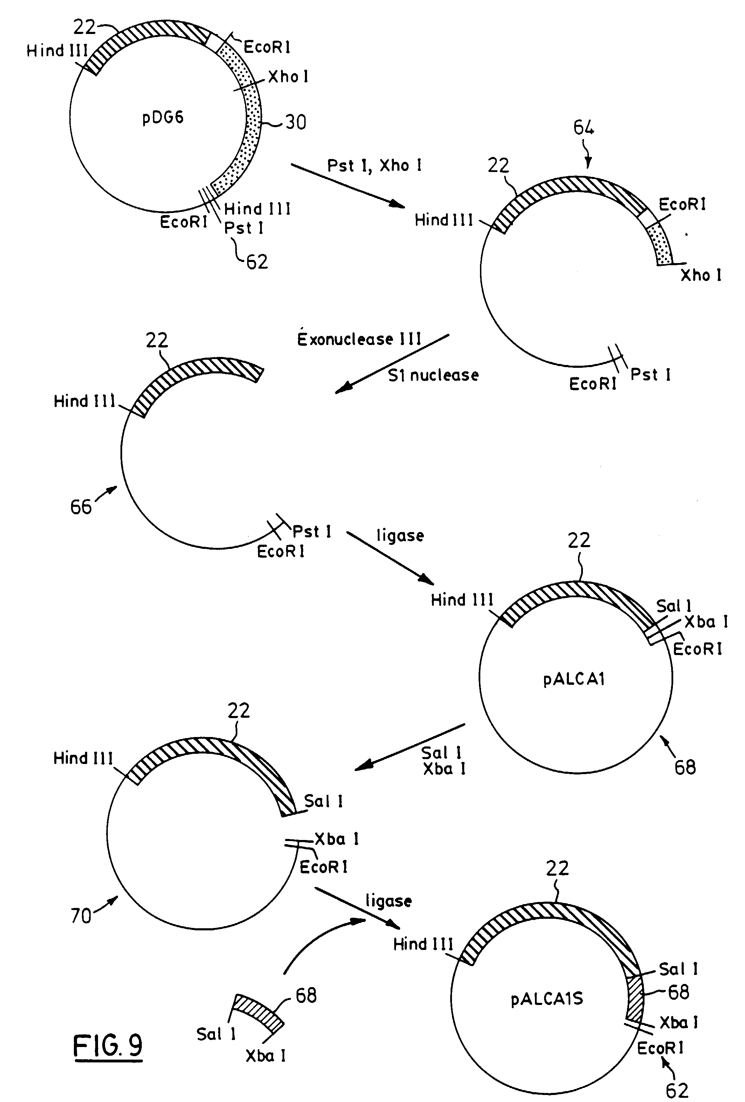

If the coding region of the vector requires a signal peptide coding region or the existing signal sequence is to be replaced by a different, preferably more efficient signal peptide coding region, such signal peptide coding regions may be integrated between the promoter and that segment coding for the secreted protein. Plasmids pGL2 (Figure 4) and pALCAlS (Figure 9) represent intermediate cloning vectors particularly suited for this purpose. Each can function as a cassette, providing a promoter, a signal sequence and a restriction site downstream of the signal sequence which permits insertion of a protein coding region in proper, transcriptional reading frame with the signal sequence.

Plasmid pGL2 shown in Figure 4 is created from pGLl which contains the promoter 40, the signal sequence 42 and an initial portion 46 of the glucoamylase gene, all of which were derived in one segment from A. niger DNA according to methods exemplified herein. In this segment, a BssHII restriction site is available toward the end of, but nevertheless within the glucoamylase signal sequence 42, the nucleotide sequence of which is reproduced below in chart 1.

Chart 1

5'   ATG TCG TTC CGA TCT CTA CTC GCC CTG AGC GGC CTC GTC TGC
     met ser phe arg ser leu leu ala leu ser gly ley val cys

                                             BssH II
     ACA GGG TTG GCA AAT GTG ATT TCC AAG CGC 3'
     thr gly leu ala asn val ile ser lys arg

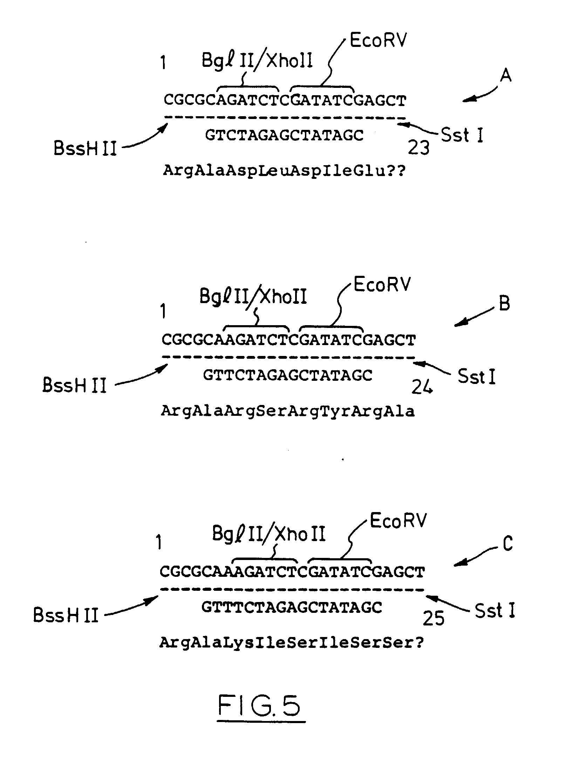

In order to provide a segment downstream of the signal sequence i.e. a linker 44, capable of receiving a protein coding region in reading frame with the signal sequence 42, advantage is taken of the presence of the BssH II site within the signal sequence and the Sst I site downstream thereof. In this specific embodiment, segment 46 is excised from pGLl and replaced with a selected one of three linkers shown in Figure 5 and denoted A, B or C. Each linker is able to ligate with the BssH II end and the Sst I end. The linkers are also engineered so as to restore the terminal codons of the signal sequence lost upon excision of segment 46 with BssH II. Further, each linker defines unique EcoRV and Bgl II/ Xho II sites within its nucleotide sequence so as to permit insertion of the desired coding region into the vector pGL2.

Selection of the appropriate linker is made with knowledge of at least the first few codons of the protein coding region to be inserted into the linker. In order for the protein coding region to be translated sensibly, the start of the protein coding region must be either directly coupled with or be a specific number of nucleotides i.e. in triplets, from the start of the signal sequence. Accordingly, if the protein coding region to be inserted possesses one or two unessential nucleotides (or a non-triplet factor thereof) at its 5' region as may result from routine excision, one of the three linkers shown in Figure 5 can compensate for the presence of the extra, superfluous nucleotides and locate the start of the protein coding region in translational reading frame with the signal sequence.

The amino acid residues encoded by the linkers A, B and C appear under their nucleotide sequences as shown in Figure 5, from which the effect of adding an additional nucleotide to the linker sequence on the reading frame of the linker and ultimately on the inserted protein encoding region may be noted. By designing the linkers such that the restriction site is always downstream of the reading frame modification i.e. one, two or three adenine residues in linkers A, B or C respectively, the reading frame of the coding region inserted into the restriction site can be maintained by appropriate linker selection.

Exemplary of plasmids which employ plasmid pGL2 and specific linker segments A, B, or C are pGL2BIFN which employs the B linker and results in interferon α-2 secretion when used in filamentous fungus e.g. Aspergillus sp., transformation and pGL2CENDO which employs the C linker and results in endoglucanase secretion when such filamentous fungi are transformed therewith.

While plasmid pGL2 utilizes a naturally occurring signal sequence, it is within the scope of the invention also to utilize vectors containing synthetic signal sequences. An example of one such vector is pALCAlS which, like plasmid pGL2, represents an intermediate vector within which a protein coding region may be inserted to form a vector capable of transforming filamentous fungi. Unlike pGL2 however, pALCAlS utilizes the alcA promoter and utilizes a synthetic signal sequence coupled to that promoter. pALCAlS is illustrated in Figure 9 which shows a scheme for preparing it and to which further reference is made in the examples. Exemplary of plasmids created from pALCAlS are pALCAlSIFN which results in secretion of interferon α-2 from a filamentous fungus transformed therewith and pALCAlSENDO which results in secretion of endoglucanase from a filamentous fungus host. In both instances secretion is obtained despite the foreign nature of the secreted protein with respect to the host.

The invention is further described and illustrated by the following specific, non-limiting examples.

Each of Examples 1 and 2 which follow exemplify successful transformation of a filamentous fungal host using vectors having a filamentous fungus-derived promoter coupled with naturally occurring but non-fungal coding regions.

### Example 1 - Transformation of A. nidulans using pDG6 ATCC 53169

The vector construct pDG6 shown in Figure 2 was first prepared following the process scheme illustrated in Figure 2, using standard routine ligation and restriction techniques. Then the construct pDG6 was introduced into Arg B⁻ mutant cells of Aspergillus nidulans as follows:

500 mls of complete media (Cove 1966) + 0.02% arginine + 10⁻⁵% biotin in a 2 1 conical flask was innoculated with 10⁵ conidia/ml of an A. nidulans Arg B⁻ strain and incubated at 30°C, shaking at 250 rpm for 20 hours. The mycelia were harvested through Whatman No. 54 filter paper, washed with sterile deionized water and sucked dry. The mycelia were added to 50 ml of filter sterile 1.2 M MgSO₄ 10 mM potassium phosphate pH 5.8 in a 250 ml flask to which was added 20 mg of Novozym 234 (Novo Enzyme Industries), 0.1 ml (=15000 units) of β-glucuronidase (Sigma) and 3 mg of Bovine serum albumin for each gram of mycelia. Digestion was allowed to proceed at 37°C with gentle shaking for 50-70 minutes checking periodically for spheroplast production by light-microscope. 50 mls of sterile deionised water was added and the spheroplasts were separated from undigested fragments by filtering through 30 um nylon mesh and harvested by centrifuging at 2500 g for 5 minutes in a swing out rotor in 50 ml conical bottom tubes, at room temperature. The spheroplasts were washed, by resuspending and centrifuging, twice in 10 mls of 0.6 M KCl. The number of spheroplasts was determined using a hemocytometer and they were resuspended at a final concentration of 10⁸/ml in 1.2 M Sorbitol, 10 mM Tris/HCl, 10 mM CaCl₂ pH 7.5. Aliquots of 0.4 ml were placed in plastic tubes to which DNA pDG6 (total vol. 40 ul in 10 mM Tris/HCl 1 mM EDTA pH 8) was added and incubated at room temperature for 25 minutes. 0.4 ml, 0.4 ml then 1.6 ml aliquots of 60% PEG4000, 10 mM Tris/HCl, 10 mM CaCl₂ pH 7.5 were added to each tube sequentially with gentle, but thorough mixing between each addition, followed by a further incubation at room temperature for 20 minutes. The transformed spheroplasts were then added to appropriately supplemented minimal media 1% agar overlays, plus or minus 0.6 M KCl at 45°C and poured immediately onto the identical (but cold) media in plates. After 3-5 days at 37°C the number of colonies growing was counted (F. Buxton et al), Gene 37, 207-214 (1985)). The method of Yelton et al [Proc. Nat'l Acad. Sci. U.S.A. 81; 1370-1374 (1980)] was also used.

The colonies were divided into two groups. Threonine (11.9 g/Liter) and fructose (1 g/Litre) were added to the incubation medium for one group to induce the cellulase gene incorporated therein. No inducer was added to the other group, which were repressed by growth on minimal media with glucose as sole carbon source. Both groups were assayed for general protein production by BioRad Assay, following cultivation, filtering to separate the mycelia, freeze drying, grinding and protein extraction with 20 mM Tris/HCl at pH 7.

To test for production of cellulase, plates of Agar medium containing cellulase (9 g/Lt, carboxymethylcellulose) were prepared, and small pieces of glass fibre filter material, isolated from one another, and 75 ug of total protein from one of the transconjugants was added to each of the filters. The plates were incubated overnight at 37°C. The filters were then removed, and the plates stained with congo red to determine the locations where cellulase had been present in the total protein on the filters, as evidenced by the breakaown of cellulase in the agar medium below. The plates were de-stained, by washing with 5M NaCl in water, to detect the differences visibly.

Of four transformants induced with threonine and fructose, three clearly showed the presence of cellulase in the total protein product. The non-induced, glucose repressed transformants did not show evidence of cellulase production.

Three control transformants were also prepared from the same vector system and strains, but omitting the promoter sequence. None of them produced cellulase, with or without inducers. The presence of C. fimi endoglucanase coding region was verified by the fact that medium from threonine-induced transformed strains showed reactivity with a monoclonal antibody raised against C. fimi endoglucanase. This monoclonal antibody showed no cross-reactivity with endogenous A. nidulans proteins in control strains.

### Example 2 - Transformation of A. nidulans using pALCAlAMY ATCC 53380

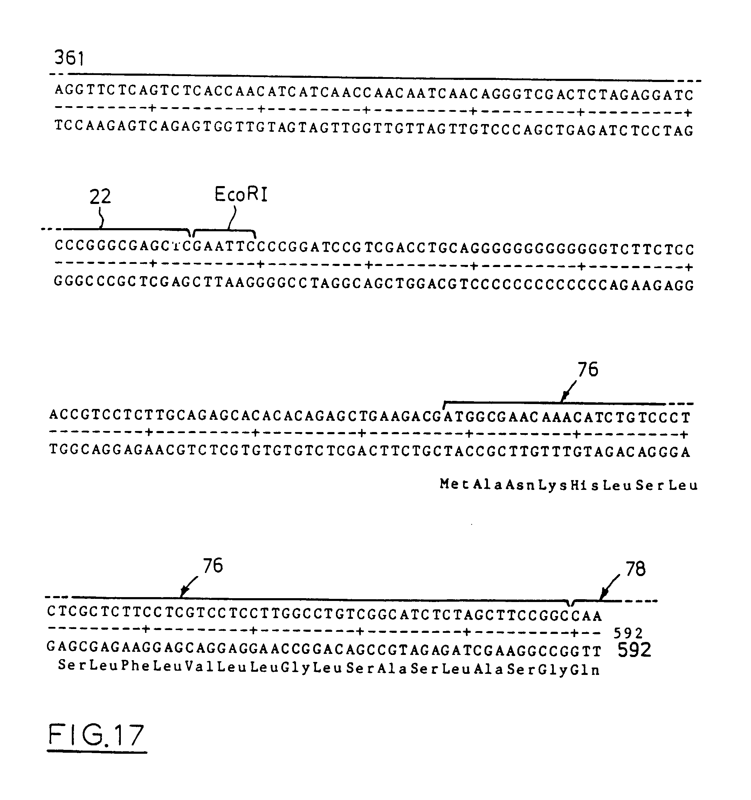

The vector construct pALCAlAMY was prepared as indicated in Figure 16, using standard routine ligation and restriction techniques. In particular, and with reference to Figure 16 vector pALCAl containing a Hind III-EcoRI segment in which the A. nidulans alcohol dehydrogenase 1 promoter 22 is located (as described previously), was cut at its EcoRI site in order to insert the coding region of the wheat α-amylase gene 72 contained within an EcoRI-EcoRI fragment defined on plasmid p501 (see S.J. Rothstein et al, Nature, 308, 662-665 (1984)). As wheat α-amylase is a naturally secreted protein, its coding region 72 contains a signal peptide coding region 76 and a segment 78 which encodes mature, secreted α-amylase. Ligation of coding region 72 contained in the EcoRI-EcoRI segment of p501 within the EcoRI-cut site of pALCAl provides plasmid pALCAlAMY in which the AlcA promoter 22 is operatively associated with the α-amylase coding region. The correct orientation of the p501-derived α-amylase coding region within pALCAlAMY is confirmed by sequencing across the ligation site according to standard procedures. The nucleotide sequence of the promoter/coding region junction is shown in Figure 17.

A. nidulans may be transformed by the procedure described in example 1, samples of extracellular medium being taken from and applied to glass fibre filter papers placed on 1% soluble starch agar. The filters are then removed after 8 hours at 37°C and inverted onto beakers containing solid iodine (in a 50°C water bath). Clear patches indicate starch degradation while the remaining starch turns a deep purple, thereby confirming the presence of secreted α-amylase.

In examples 3-12 which follow, vectors are provided in which a secretion signal peptide coding region is introduced in the vector in order to obtain secretion of a foreign protein from a filamentous fungus transformed by the entire vector.

### Example 3 - Production of Plasmid pGL2, an intermediate vector

### A) Source of promoter and signal peptide sequence

The glucoamylase gene of A. niger was isolated by probing a gene bank derived from DNA available in a strain of this microorganism on deposit with ATCC under catalogue number 22343. The probing was conducted using oligonucleotide probes prepared with Biosearch oligonucleotide synthesis equipment and with knowledge of the published amino acid sequence of the glucoamylase protein. The amino acid sequence data was "reverse translated" to nucleotide sequence data and the probes synthesized. The particular gene bank probed was a Sau 3A partial digest of the A. niger DNA described above cloned into the Bam HI site of the commercially available plasmid pUC12 which is both viable in and replicable in E. Coli.

A Hind III -Bgl II piece of DNA containing the glucoamylase gene was subcloned into pUC12. Subsequently, the location of the desired promoter region, signal peptide coding region and protein coding region of the glucoamylase was identified within pUC12 containing the sub-cloned fragment. The EcoRI/EcoRI fragment (see Figure 4) was shown to contain a long, open translation reading frame when it was sequenced and the sequence data was analyzed using the University of Wisconsin sequence analysis programmes.

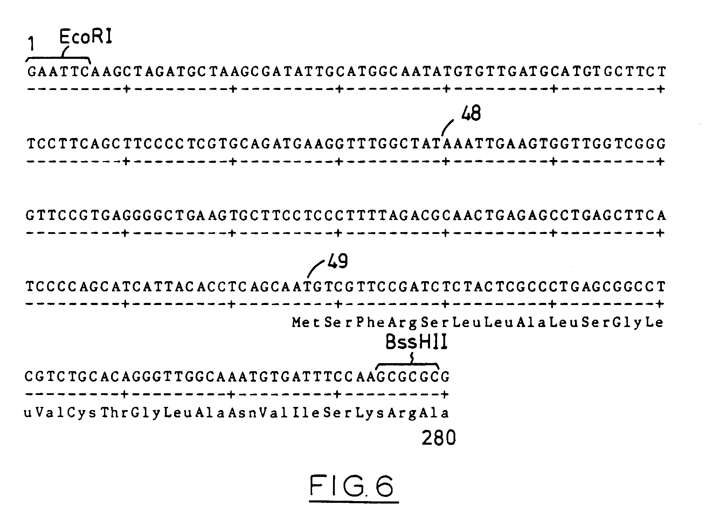

Results of analysis of the nucleotide sequence of part of the region of the glucoamylase gene between the 5' Eco RI site and BssH II 3' site within the Hind III - Bgl II fragment are shown in Figure 6. This region contains the glucoamylase promoter and the signal peptide coding region.

Within this fragment i.e. at nucleotides 97-102 is a "TATA box" 48 which provides a site required by many eukaryotic promoter regions for accurate initiation of transcription (probably an RNA polymerase II binding site). Accordingly, the presence of at least a portion of the promoter region is confirmed. Further, it is predictable from analogy with other known promoter regions that all the functional essentials are likely to be contained within a sequence of about 1,000 bases in length and most likely within the first 200 - bases upstream of the start codon for the coding region i.e. nucleotides 206 - 208 or "ATG" 49, the codon for methionine. Thus, the promoter and transcript leader terminate at nucleotide 205. The identity of the beginning of the promoter region is less crucial although the promoter region must contain the RNA polymerase II binding site and all other features required for its function. Thus, whereas the Eco RI-Eco RI sequence is believed to represent the entire promoter region of the glucaomylase gene, the fragment used in plasmid pGL2 contains this fragment in the much larger Hind III - BamH I/Bgl II segment to ensure that the entire promoter region is properly included in the resultant plasmid.

On the basis that the amino acid sequence of mature glucoamylase is known (see Svensson et al, "Characterization of two forms of glucoamylase from Aspergillus niger", Carlsberg Res. Commun, 47, 55-69 (1982)), a nucleotide sequence ofthe signal peptide can be determined accurately. The signal peptide coding region of genes encoding secreted proteins is known to initiate with the methionine residue encoded by the ATG codon 49. Determination of a sufficient initial portion of the nucleotide sequence beyond i.e. 3' of the ATG codon provides information from which the amino acid sequence of that portion may be determined. By comparison of this amino acid sequence with the published amino acid sequence, the signal peptide can be identified as that portion of the glucoamylase gene which has no counterpart in the published sequence with which it was compared. The glucoamylase signal peptide coding region defined herein was previously confirmed using this method.

By the above methods, the Hind III - Bam HI/Bgl II fragment resulting from Sau 3A partial digestion and incorporated into pUC12 was confirmed to contain the following features of the glucoamylase gene: an initial, perhaps non-relevant section, the promoter region, the signal peptide coding region and the remaining portion of the coding region. This fragment, inserted into the pUC12 plasmid by scission with Hind III and Bam HI/Bgl II and ligation appears schematically in Figure 4 as plasmid pGLl. This plasmid contains all of the features necessary for replication and the like in order to remain selectable and replicable in E. Coli.

### B) Construction of Plasmid pGL2

Using pGLl as a precursor, plasmid vector pGL2 can be formed as shown in Figure 4. The restriction site BssH II near the 3' end of the signal sequence 42, is utilized together with the unique downstream Sst I site in order to insert a synthetic linker sequence A, B, or C defined in Figure 5 herein. Thus, pGLl is cleaved with both BssH II and Sst I thereby removing the initial portion of the glucoamylase coding region 46 contained therein. Thereafter a selected one of the synthetic leader sequences A through C having been designed so as to be flanked by BssH II/Sst I compatible ends is inserted and ligated, thereby generating plasmid pGL2. Depending on which of the three linker sequences is used i.e. A, B or C, the resultant plasmid will hereinafter be identified as pGL2A, pGL2B or pGL2C, respectively.

The synthetic linker sequences identified herein are each equipped with unique Eco RV and Bgl II restriction sites, as shown in Figure 5, into which a desired protein coding region may be inserted. Once inserted, the resultant plasmid may be used to transform a host e.g. A niger, A. nidulans and the like. The presence of the promoter region and the signal peptide coding region both of which are recognized by the host, provide a means whereby expression of the protein coding region and secretion of the protein so expressed is made possible.

### Example 4 - Use of Plasmid pGL2 in creating pGL2BIFN

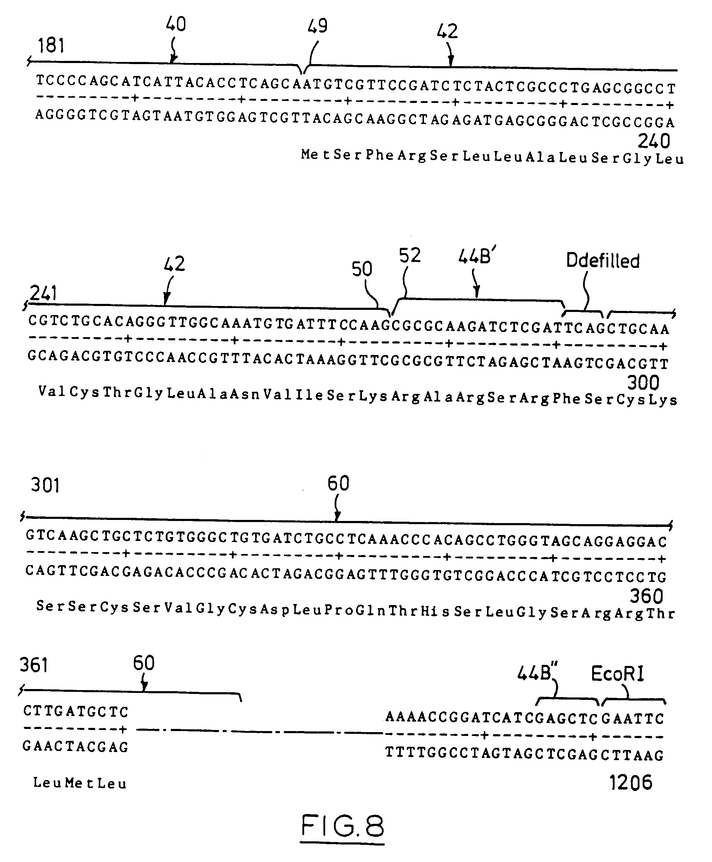

An example of the utility of the plasmid pGL2 is described below with reference to Figure 7, which shows schematically the construction of plasmid pGL2BIFN from pGL2B.

The plasmid pGL2B is prepared as described in general previously for pGL2 save that synthetic linker sequence "B" shown in Figure 5 is inserted specifically. The reference numeral 44 has accordingly been modified in Figure 7 to read "44B". In order to make available an opening in the vector pGL2B, the plasmid is cut with Eco RV at the site internal to linker 44B. The scission results in blunt ends which may be ligated with a fragment flanked by blunt ends using ligases known to be useful for this specific purpose.

In the embodiment depicted in Figure 7, a fragment 60 containing the coding region of human interferon α-2 is inserted to create pGL2BIFN. Specifically, a Dde I - Bam HI fragment 60 containing the coding region coding for human interferon α- 2 was excised from plasmid pN5H8 (not shown) on the basis of the known sequence and restriction map of this gene.

The plasmid pN5H8 combines known plasmid pAT153 with the interferon gene at a Bam HI site. The interferon gene therein is described by Slocomb, et. al., "High level expression of an interferon α-2 gene cloned in phage M13mp7 and subsequent purification with a monoclonal antibody" Proceedings of the National Academy of Sciences, U.S.A., Vo. 79 pp 5455-5459 (1982)

In order to anneal the sticky ends of the interferon fragment into the cut Eco RV site of pGL2B, the sticky Dde I and Bam HI ends are filled using reverse transcriptase and ligated with an appropriate ligase according to techniques standard in the art.

The advantage of selecting linker sequence B for insertion into pGL2 is manifest from Figure 8 which shows the reading frame of the interferon α-2 coding region and its relationship with the recreated signal peptide sequence, in terms of nucleotide sequence and amino acid sequence, where appropriate.

Figure 8 shows a portion of the promoter region 40 5' of the signal sequence joined with a portion of the glucoamylase signal peptide sequence 42 beginning with the methionine codon ATG at 49 and ending with the lysine codon AAG at 50. In fact, although the signal peptide coding region extends one residue further i.e. to the CGC codon for arginine at 52, this latter residue is comprised by the synthetic linker sequence 44B engineered so as to compensate for the loss of the arginine residue during scission and ligation to insert the linker sequence. In this way, the genetic sequence of the signal remains undisturbed.

In a similar manner, the linker sequence provides for insertion of the interferon α-2 coding region without altering the reading frame thereof. With reference to Figures 7 and 8 cleavage of linker sequence 44B by Eco RV results in linker fragments 44B' and 44B" having blunt ends. Excision of the interferon α-2 coding region at Dde I site results, after filling in of the sticky ends created by the enzyme, in the desired nucleotide sequence without harming the sequence of that coding region. Ligation within the Eco RV-cleaved linker sequence of the interferon sequence filled at the Dde I site maintains the natural reading frame of the interferon coding region as evidenced by the triplet codon state between the linker portion 44B' and the interferon coding region 60. Had the linker A shown in Figure 5 been chosen, which bears one less nucleotide than the linker B, the entire reading frame would have been shifted by one nucleotide resulting in a nonsense sequence. By selection of synthetic linker B, codons are made available between the signal peptide sequence and the interferon coding region which do not alter the reading frame of the coding region, when the blunt ended lF α-2 fragment is oriented correctly. The correct orientation is selected by sequencing clones with inserts across the ligation junction.

### Example 5 - Expression and Secretion from A. nidulans Transformed with pGL2BIFN ATCC 53371

The plasmid pGL2BIFN was cotransformed i.e. with a plasmid containing Arg B⁺ gene as described more fully in U.S. patent application serial No. 678,578 filed December 5, 1984 into an Arg B⁻ strain of A. nidulans with a separate plasmid containing an arg B selectable marker. Arg B⁺ transformants were selected of which 18 of 20 contained 1 - 100 copies of the human interferon α-2 coding region (as detected by Southern blot analysis).

Several transformants were grown on starch medium to induce the glucoamylase promoter and the extracellular medium was assayed for human lF α-2 using the CellTech lF α-2 assay kit.

All transformants exhibited some level of synthesis and secretion of assayable protein. Two controls, the host strain (not transformed) and one arg B⁺ transformant with no detectable human lF α-2 DNA showed no detectable synthesis of lF α-2 protein. In a separate experiment, transformation of A. niger, rather than A. nidulans, with pGL2BlFN using, mutatis mutandis, the same procedure as described above, demonstrated the ability of A. niger to secrete lF α-2.

Thus, although the promoter and signal regions of pGL2BIFN are derived from A. niger they are shown to be operative in both A. nidulans and A. niger.

In the present invention, use may be made of promoter regions other than the glucoamylase promoter region. Suitable for use are the promoter regions of the alcohol dehydrogenase I gene and the aldehyde dehydrogenase gene, illustrated in Figures 1A and 1B.

### Example 6 - Construction of Plasmid pALCAlS, ATCC 53368 an intermediate vector

For use with the present example, the alcA promoter was employed as comprised within an 10.3 kb plasmid pDG6 deposited with ATCC within host E. Coli JM83 under accession number 53169. A plasmid map of pDG6 is shown in Figure 2 and, for ease of reference, in Figure 9 to which reference is now made, to illustrate another embodiment using the alcA promoter.

pDG6 comprises, in its Hind III-EcoRI (first occurrance) segment, the promoter region 22 of the alcA gene as well as a small 5' portion of the alcA coding region 3' of the start codon, ligated to the endoglucanase coding region 30. pDG6 further comprises a multiple cloning site 62 downstream of the C. fimi endoglucanase coding region 20.

To retrieve the alcA promoter region 22, pDG6 was cut with Pst I and Xho I removing the bulk of the endoglucanase coding region 30. In a second step, the linearized plasmid 64 was resected in one direction in a controlled manner with exonuclease III (which will resect from XhoI but not PstI-cut DNA ends) followed by tailoring with nuclease S1. The resection was timed so that the enzyme removed nucleotides to a position 50 bases 5' of the alcA ATG codon, leaving the TATA box and messenger RNA start site intact.

Following resection, the vector 66 was religated (recircularized) creating vector 68 bearing Sal I-Xba I restriction sites immediately downstream of the promoter region 22. Cleavage of vector 68 with Sal I/Xba I permits introduction of a signal peptide coding region at an appropriate location within the vector.

The particular signal peptide coding region employed in the present example was synthesized to reproduce a characteristic signal peptide coding region identified according to standard procedures as described by G. Von Heijne in Eur. J. Biochem. 17-21, (1983). The synthetic signal was engineered so as to provide a 5' flanking sequence complementary to a Sal I cleavage site and a 3' flanking sequence enabling ligation with the Xba I restriction sequence.

The sequence of the synthetic secretion signal 68 is reproduced below:

Sal I

TCGACATGTACCGGTTCCTCGCCGTCATCTCGGCCTTCCTCGCCACTGCCTTCGCCAAG
1 ---------+---------+---------+---------+---------+---------+ 59
GTACATGGCCAAGGAGCGGCAGTAGAGCCGGAAGGAGCGGTGACGGAAGCGGTTC

MetTyrArgPheLeuAlaValIleSerAlaPheLeuAlaThrAlaPheAlaLys

Xba I

T
60 ------ 64
AGATC

SerArg

The secretion signal per se begins with Met and ends with the fourth occurrence of Ala, as indicated by the arrow.

Once generated, the synthetic sequence 68 acting as signal is cloned into the Sal I-Xba I site of vector 70 resulting in plasmid pALCAIS which contains alcA promoter region 22, and synthetic peptide signal coding region 68. That the signal peptide coding region is inserted upstream of the multiple cloning site 62 is significant in that the site 62 allows for cloning of a variety of protein coding segments within this plasmid.

Accordingly, pALCAlS constitutes a valuaDle embodiment of the present invention.

### Example 7 - Construction of Plasmid pALCAlSIFN

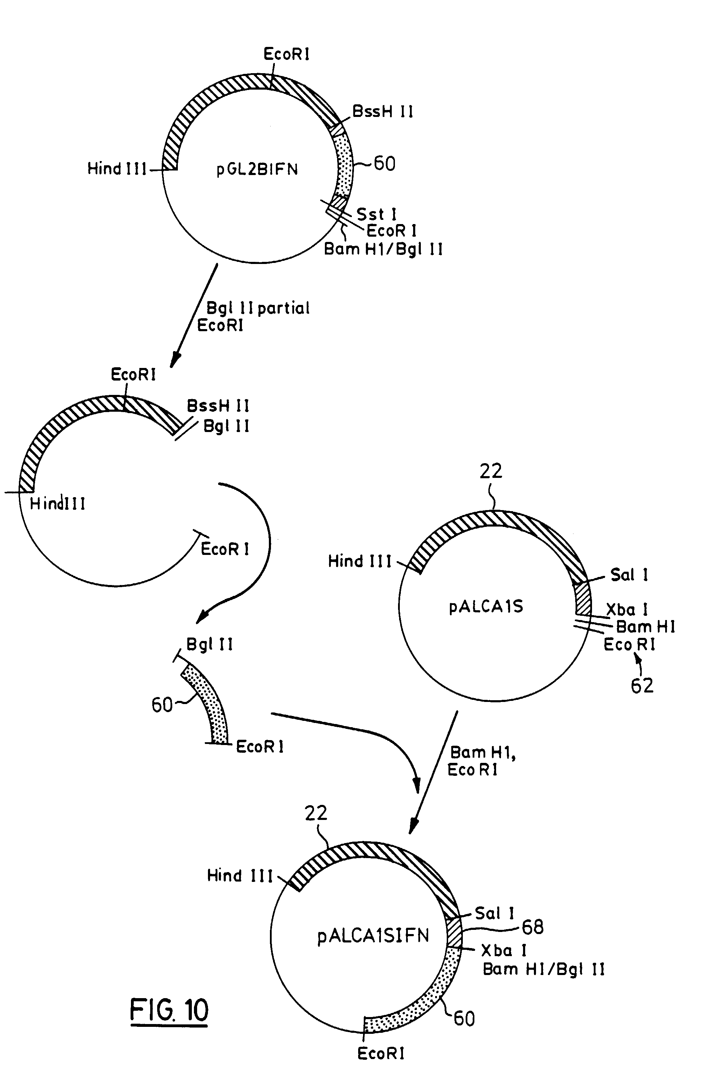

As an example of the utility of pALCAlS, reference is made to Figure 10 showing creation of pALCAlSIFN. This plasmid comprises the promoter region 22 of the alcA gene and the synthetic signal peptide coding region 68 both of which are derived from pALCAlS (Figure 9). In addition, it contains the coding region 60 coding for human interferon α-2 derived from pGL2BIFN.

To obtain the protein encoding segment, pGL2BIFN is cleaved with Eco RI and partially cleaved with Bgl II (because of the presence of internal Bgl II sites). Insertion of the protein coding region is accomplished by cleaving pALCAIS with Bam HI and Eco RI both of which are available in the multiple cloning site 62 and ligating this coding region therein, thereby creating pALCAlSIFN.

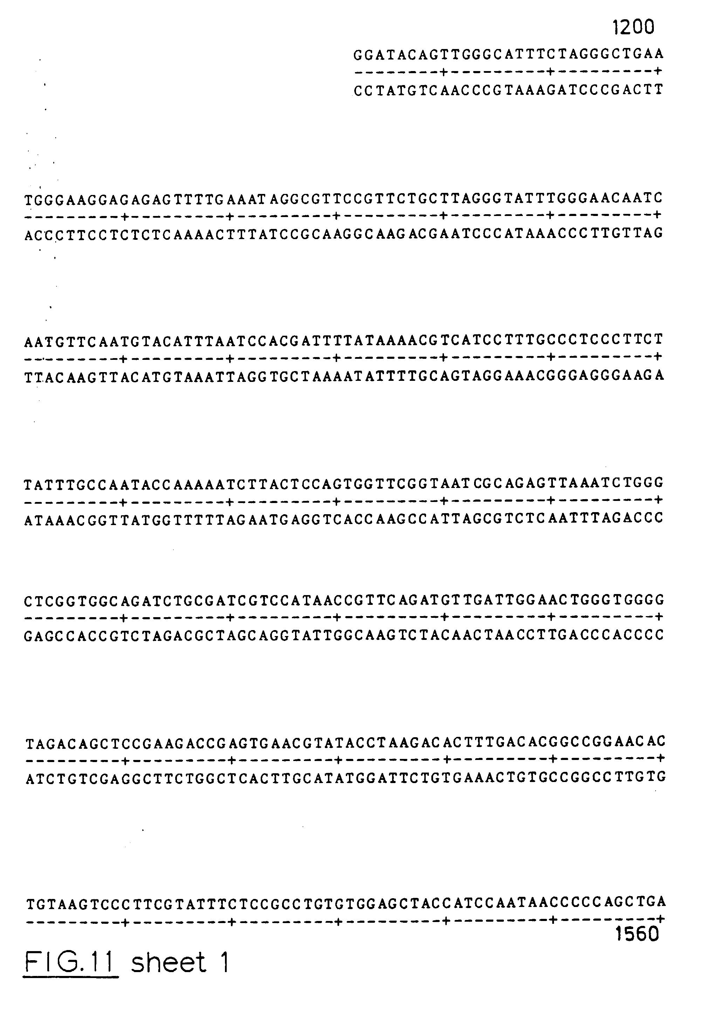

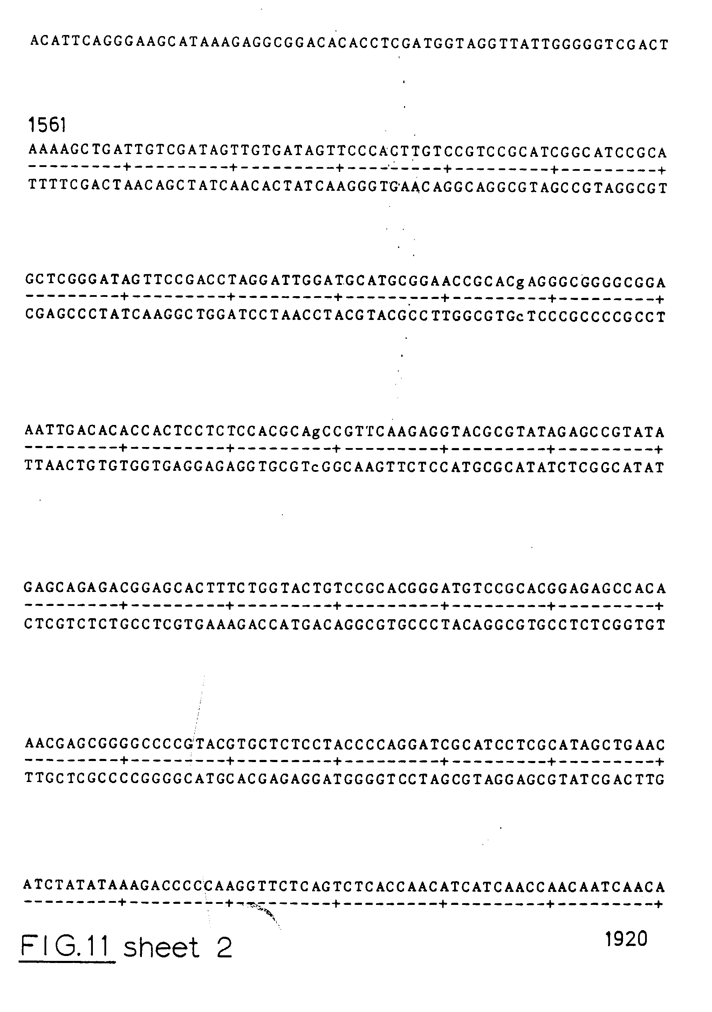

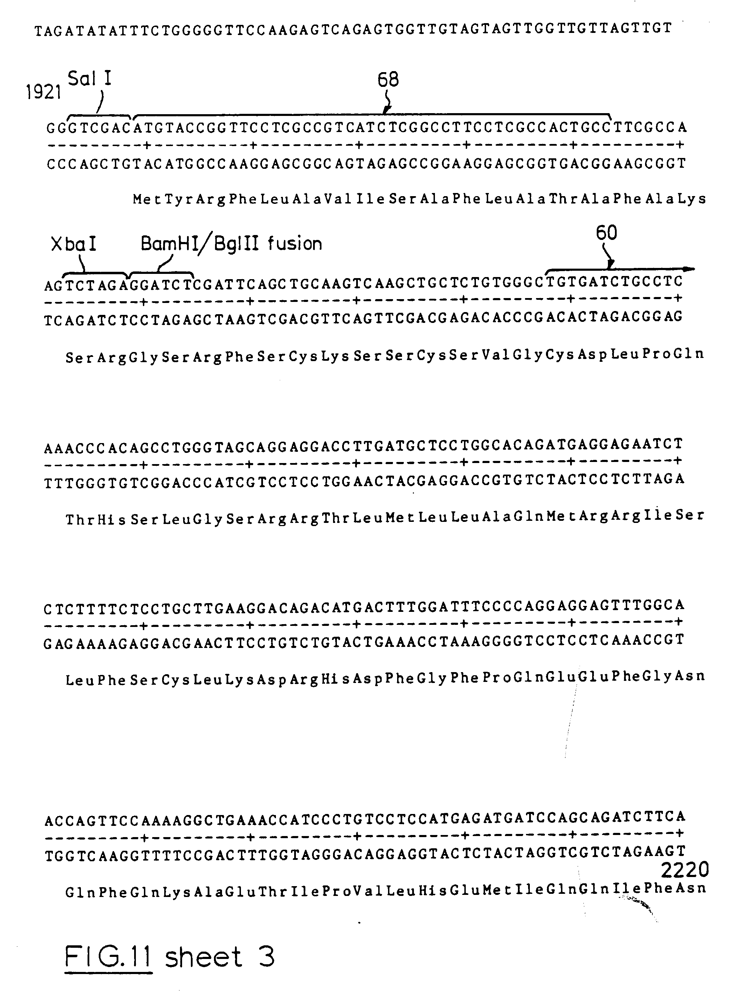

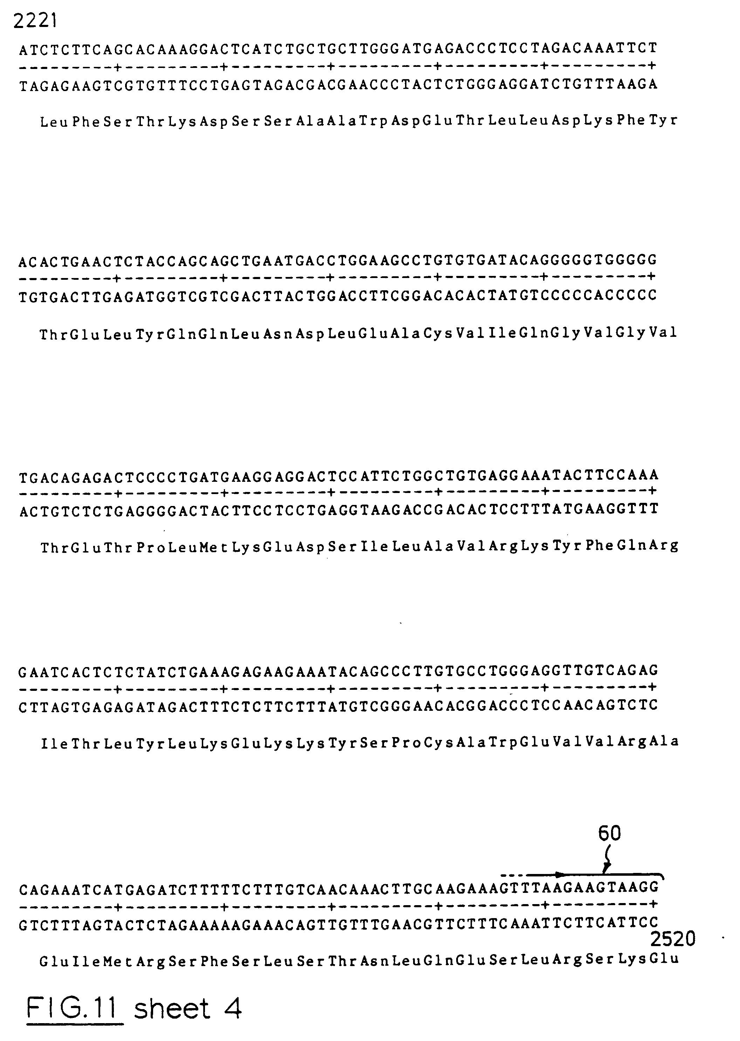

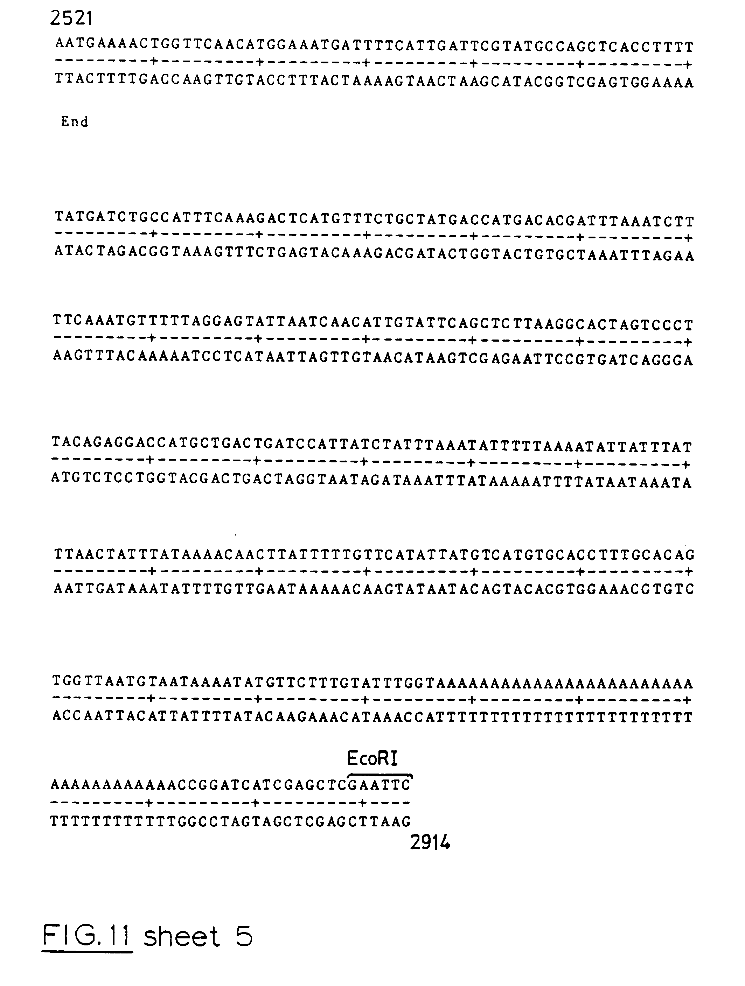

The nucleotide sequence of the resultant plasmid, from a site 1170 nucleotides downstream of Hind III ta Eco RI is shown in Figure 11, indicating the relevant sites of restriction endonuclease digestion. It will be noted from sheet 3 of Figure 11 that the lF α-2 coding region 60 is in proper reading frame with the synthetic signal peptide coding region 68.

### Example 8 - Expression and Secretion from A. Nidulans Transformed with Plasmid ALCAlSIFN

The plasmid pALCAlSIFN prepared as described above was co-transformed with A. nidulans to provide an arg B selectable marker, the arg B⁺ transformants selected and checked for the presence of the human interferon α-2 coding region, then grown on a threonine-containing medium to induce the alcA promoter, all as described in example 3 above. The extracellular medium was assayed for human IF-2 using Cell Tech IFα2 assay kit. Eleven of twenty transformants showed secretion of interferon, induced in the presence of threonine, and repressed in the presence of glucose.

### Example 9 - pGL2CENDO ATCC 53372

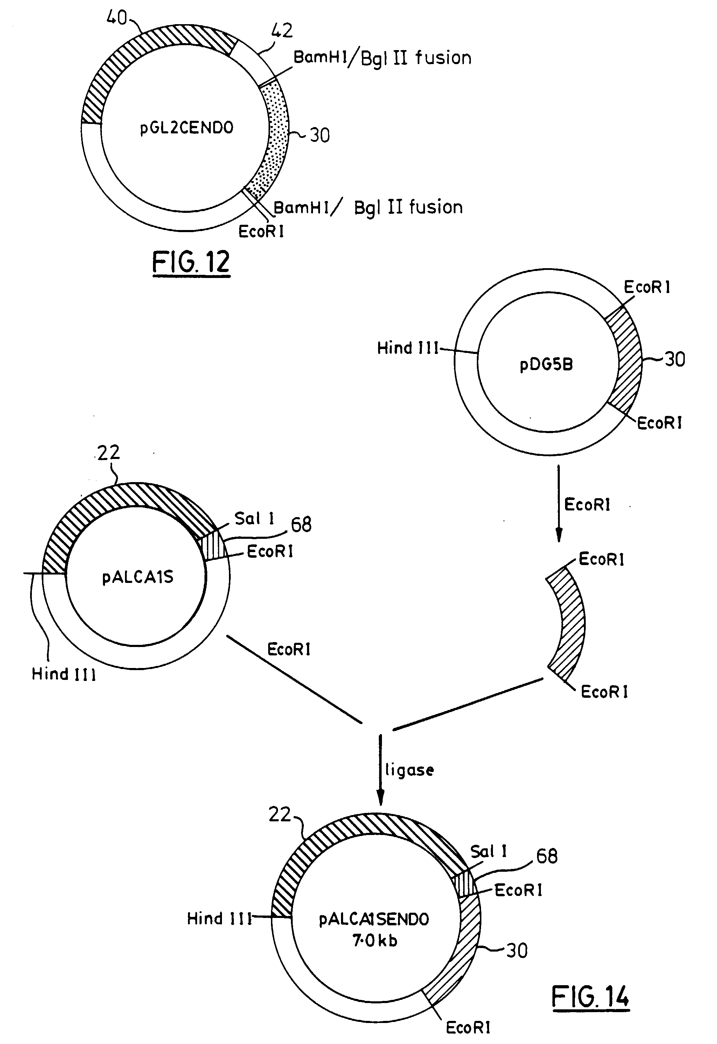

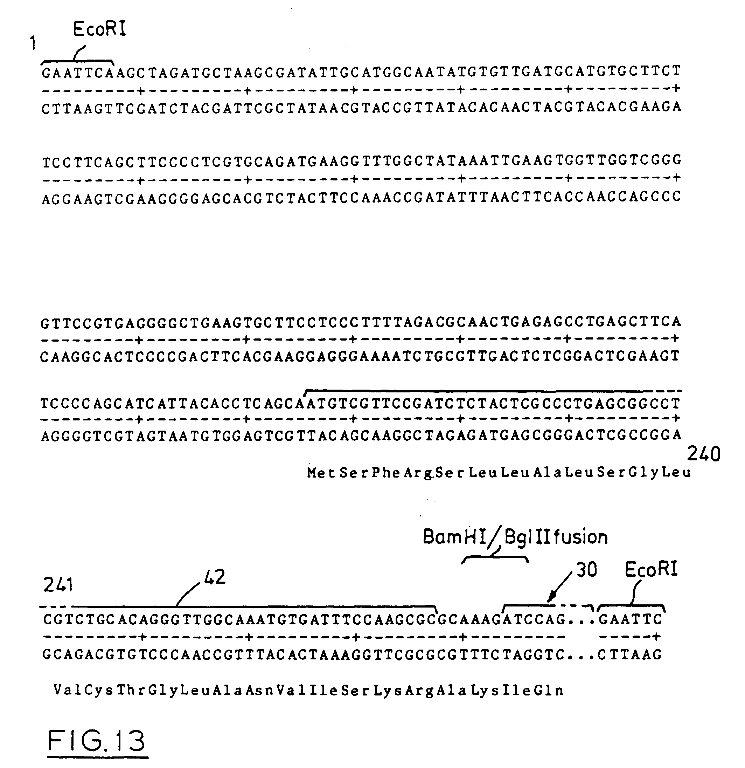

In accordance with the procedures described in the previous examples, there was constructed a vector plasmid designated pGL2CENDO, from plasmid pGL2C ATCC 53367, analagous to pGL2BIFN shown in Fig. 7, but containing the endoglucanase coding region in place of the interferon 2 coding region, and using the synthetic linker sequence "C" (Fig. 5) in place of linker sequence "B". A Bam HI fragment containing the C. fimi endogluconase coding region 30 was inserted into the Bgl II site of pGL2C. A. nidulans transformants were prepared with this vector plasmid, and showed starch regulated secretion of cellulase assayed as described in Example 1. The map of vector plasmid pGL2CENDO is shown in Fig. 12 of the accompanying drawings, in which 30 denotes the endoglucanase coding region (the endoglucanase coding region of Cellulomonas fimi, described in connection with Fig. 2 and Example 1), 42 denotes the signal peptide coding region of the glucoamylase gene and 40 denotes the promoter region of the glucoamylase gene. The nucleotide sequence is shown in Figure 13 and exemplifies that use of linker sequence C (Fig. 5) retains the reading frame of the signal peptide coding region 42 and the endoglucanase coding region 30.

### Example 10 - Construction of Plasmid pALCAlSENDO ATCC 53370

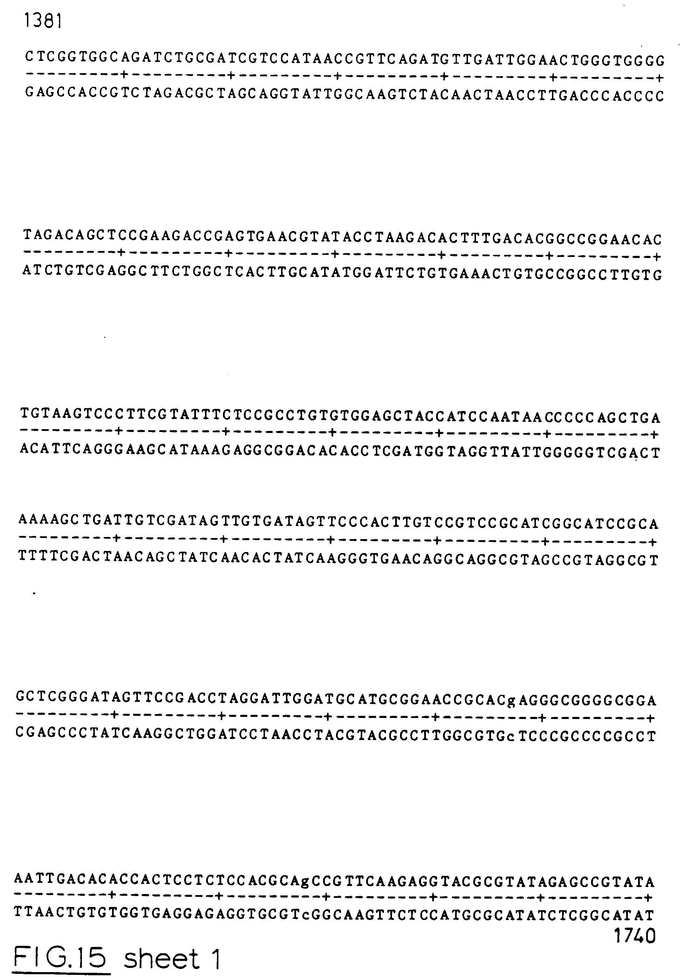

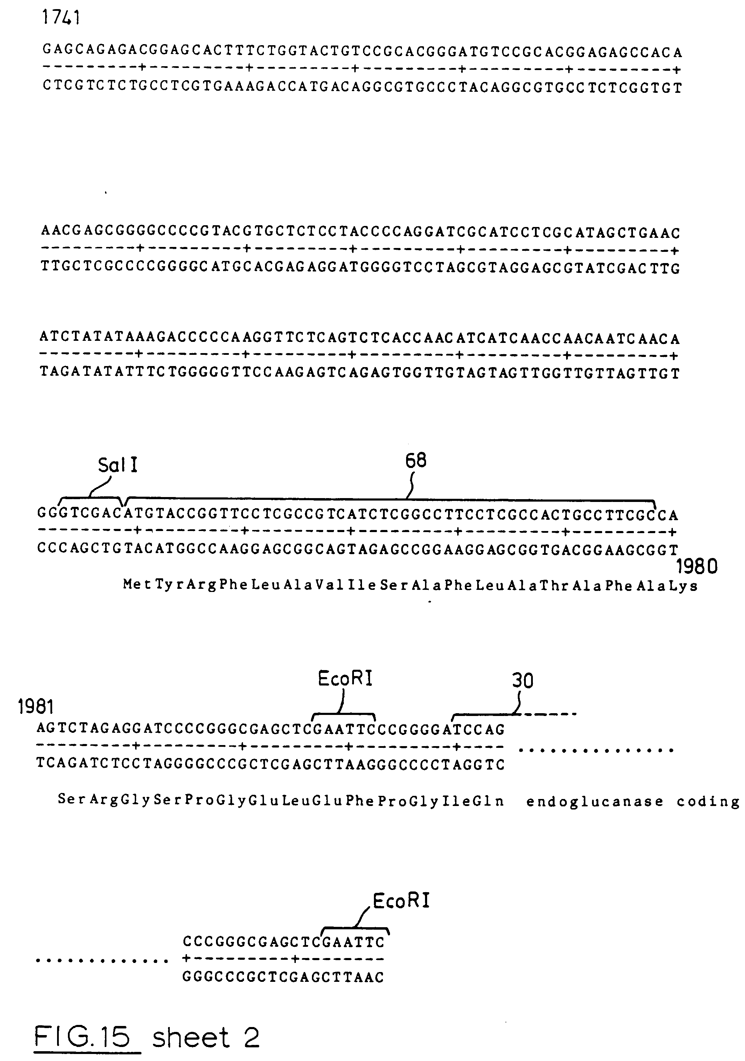

In accordance with the procedures described in the previous examples, there was constructed a vector plasmid designated pALCAlSENDO by combining Eco RI - linearized plasmid pALCAlS as described in example 5 (Fig. 9) with an Eco RI fragment derived from plasmid pDG5B (see Fig. 2) (pDG5 with the orientation of the Hind III fragment reversed in pUC12) and containing the endoglucanase coding region 30. The map of pALCAlSENDO is shown in Figure 14 and the nucleotide sequence of its pertinent region is shown in Figure 15. In these figures, the promoter region derived from alcA is designated by numeral 22, the synthetic signal peptide coding region is designated 68 and the endoglucanase coding region is designated by reference numeral 30.

### Example 11 - Expression and Secretion from A. nidulans Transformed with pALCAlSENDO and pGL2CENDO

A. nidulans was co-transformed with an argB+ selectable marker and the plasmid pALCAlSENDO or pGL2CENDO prepared as described above. Of the co-transformants obtained several showed varying levels of secretion of cellulase (i.e. endoglucanase) as assayed on carboxymethylcellulose plates and the monoclonal antibody test systems as described in example 1. Both plasmid transformants showed secretion which was controlled by the linked promoter. Plasmid pGL2CENDO was induced by starch and pALCAlSENDO was induced with threonine.

### Example 12 - Expression and Secretion From A. niger Transformed with pGL2CENDO

A. niger was cotransformed with an argB+ selectable marker and the plasmid pGL2CENDO. Several of the transformants showed varying levels of secretion of endoglucanase as assayed as described in example 1. This secretion was induced by the presence of starch in the medium.

### Example 13 - Increased Copy Number of Regulatory Genes

In Aspergillus nidulans the alcA promoter is turned on in the presence of the appropriate inducer, such as ethanol, by the action of the gene product of alcR, the positive regulatory gene for alcA.

Evidence with multiple copy transformants (containing multiple alcA promoters) suggests that the alcR gene product limits the promoter function of the several alcA promoters requiring stimulation.

Increasing the copy number of the alcR gene increases the expression of alcR and relieves this situation. The evidence for this is as follows:

Transformants with multiple copies of the alcA promoter fused to its own coding region (ADH I) in a multiple alcR background (which has been shown to overproduce alcR messenger RNA) do not grow well on ethanol. This is probably due to rapid accumulation of aldehydes, the product of ADH breakdown of ethanol. ADH activity in these strains is high. The increased activity of ADH due to increased copy number probably accounts for these observations.

Transformants with multiple copies of the alcA promoter fused to interferon α-2 in a multiple alcR background produce significantly higher levels of secreted interferon. In these strains, unlike those with single copy alcR, many more of the alcA promoters have access to the alcR regulatory protein.

Thus, preferred embodiments of the present invention provide means for introducing a coding region into a filamentous fungus host which, when transformed, will secrete the desired protein. Particularly useful intermediate plasmids for this purpose are pALCAlS and pGL2 (A, B or C).

Useful transformation vectors created from these plasmids include pALCAlSIFN, pGL2BIFN, pALCAlSENDO and pGL2CENDO. Cultures of each of these and other plasmids mentioned herein are currently maintained in a permanently viable state at the laboratories of Allelix Inc., 6850 Goreway Drive, Mississauga, Ontario, Canada. The plasmids will be maintained in this condition throughout the pendency of this patent application and, during that time, will be made available to authorized persons. After issue of a patent on this application, these plasmids will be available from the ATCC depository recognized under the Budapest Treaty, without restriction. The accession numbers of the respective deposits appear in the table below:

| Plasmid | Host | Accession # | Deposit Date |
|---|---|---|---|
| | | | |
| pDG6 | E. Coli JM83 | 53169 | June 7, 1985 |
| pGL2A | " | 53365 | Dec. 16, 1985 |
| pGL2B | " | 53366 | " |
| pGL2C | " | 53367 | " |
| pALCAlS | " | 53368 | " |
| pALCAlSENDO | " | 53370 | " |
| pALCAlSIFN | " | 53369 | " |
| pGL2BlFN | " | 53371 | " |
| pGL2CENDO | " | 53372 | " |
| pALCAlAMY | " | 53380 | Dec. 20, 1985 |

## Claims

1. A method for producing a polypeptide which comprises:
(i) culturing a filamentous fungus which has been transformed by a recombinant DNA construct in which a regulatable promoter region of a filamentous fungus gene is operably linked to a DNA fragment encoding the polypeptide, wherein:
(a) said DNA fragment is foreign to the promoter region; and
(b) said regulatable promoter region is inducible by ethanol said ethanol inducibility being mediated by the alcR gene product;
(ii) under conditions in which regulated expression of the polypeptide occurs; and
(iii) recovering said polypeptide.

2. A method according to claim 1 wherein the promoter region is repressible by glucose.

3. A method according to claim 2 which comprises culturing the filamentous fungus in the presence of glucose during the cell growth phase and adding an inducer in the absence of glucose to enhance the activity of the promoter region.

4. A method according to any one of the preceding claims wherein the promoter region is derived from an alcohol dehydrogenase gene or an aldehyde dehydrogenase gene of a filamentous fungus.

5. A method according to any one of the preceding claims wherein the DNA construct containing the regulatable promoter region is present in multiple copies.

6. A method according to claim 5 wherein the filamentous fungal strain expresses greater than normal levels of alcR, thereby increasing the level of expression of the multiple copies of the DNA construct containing the regulatable promoter region.

7. A method according to any one of claims 1 to 6, wherein the regulatable promoter region is derived from an Aspergillus gene.

8. A method according to claim 7, wherein the regulatable promoter region is derived from an Aspergillus nidulans gene.

9. A method according to claim 7, wherein the regulatable promoter region is derived from an Aspergillus niger gene.

10. A method according to any one of the preceding claims wherein the filamentous fungus is an Aspergillus.

11. A method according to claim 10 wherein said Aspergillus is A.nidulans.

12. A method according to claim 10 wherein said Aspergillus is A.niger.

13. A filamentous fungus as defined in any one of claims 1, 2 or 4 to 12.

14. A recombinant DNA construct comprising a regulatable promoter region of a filamentous fungus gene operably linked to a DNA fragment encoding the polypeptide, wherein:
(a) said DNA fragment is foreign to the promoter region; and
(b) said regulatable promoter region is inducible by ethanol said ethanol inducibility being mediated by the alcR gene product.

15. A recombinant DNA construct according to claim 14 which is as further defined in any one of claims 2 or 4 to 12.

## Patentansprüche

1. Verfahren zur Harstellung eines Polypeptids, umfassend:
(i) das Züchten eines filamentösen Pilzes, der durch ein rekombinantes DNA-Konstrukt transformiert ist, wobei in dem Konstrukt eine regulierbare Promotorregion eines filamentösen Filzgens funktionell mit einem für das Polypeptid kodierenden DNA-Fragment verknüpft ist. wobei:
(a) das DNA-Fragment für die Promotorregion fremd ist; und
(b) die regulierbare Promotorregion durch Ethanol induzierbar ist, wobei die Ethanol-Induzierbarkeit durch das alcR-Genprodukt vermittelt wird;
(ii) unter Bedingungen, bei denen die regulierte Expression des Polypeptids erfolgt; und
(iii) das Gewinnen des Polypeptids.

2. Verfahren nach Anspruch 1, wobei die Promotorregion durch Glucose reprimierbar ist.

3. Verfahren nach Anspruch 2, umfassend das Züchten des filamentösen Pilzes in Gegenwart von Glucose während der Zellwachstumsphase und das Zusetzen eines Inducers in Abwesenheit von Glucose, um die Aktivität der Promotorregion zu verstärken.

4. Verfahren nach einem der vorstehenden Ansprüche, wobei die Promotorregion von einem Alkohol-dehydrogenase-Gen oder einem Aldehyddehydrogenase-Gen eines filamentösen Pilzes abgeleitet ist.

5. Verfahren nach einem der vorstehenden Ansprüche, wobei das DNA-Konstrukt, das die regulierbare Promotorregion enthält, in mehrfachen Kopien vorhanden ist.

6. Verfahren nach Anspruch 5, wobei der filamentöse Pilzstamm das alcR über das normale Niveau hinaus exprimiert, wodurch das Expressionsniveau der mehrfachen Kopien des DNA-Konstrukts, das die regulierbare Promotorregion enthält, erhöht wird.

7. Verfahren nach einem der Ansprüche 1 bis 6, wobei die regulierbare Promotorregion von einem Aspergillus-Gen abgeleitet ist.

8. Verfahren nach Anspruch 7, wobei die regulierbare Promotorregion von einem Aspergillus nidulans-Gen abgeleitet ist.

9. Verfahren nach Anspruch 7, wobei die regulierbare Promotorregion von einem Aspergillus niger-Gen abgeleitet ist.

10. Verfahren nach einem der vorstehenden Ansprüche, wobei as sich beim filamentösen Pilz um einen Aspergillus-Pilz handelt.

11. Verfahren nach Anspruch 10, wobei es sich beim Aspergillus-Pilz um A. nidulans handelt.

12. Verfahren nach Anspruch 10, wobei es sich beim Aspergillus-Pilz um A. niger handelt.

13. Filamentöser Pilz gemäß der Definition in einem der Ansprüche 1, 2 oder 6 bis 12.

14. Rekombinantes DNA-Konstrukt, umfassend eine regulierbare Promotorregion eines filamentösen Pilzgens, das operativ mit einem für das Polypeptid kodierenden DNA-Fragment verknüpft ist, wobei:
(a) das DNA-Fragment für die Promotorregion fremd ist; und
(b) die regulierbare Promotorregion durch Ethanol induzierbar ist, wobei die Ethanol-Induzierbarkeit durch das alcR-Genprodukt vermittelt wird;

15. Rekombinantes DNA-Konstrukt nach Anspruch 14, das zusätzlich der Definition in einem der Ansprüche 2 oder 4 bis 12 entspricht.

## Revendications

1. Procédé de production d'un polypeptide, comprenant :
(i) la culture d'un champignon filamenteux qui a été transformé par une construction d'ADN recombinant, dans laquelle un promoteur régulable d'un gène de champignon filamenteux est lié de façon opérante à un fragment d'ADN codant pour le polypeptide, dans laquelle :
(a) ledit fragment d'ADN est étranger au promoteur, et
(b) ledit promoteur régulable peut être induit par l'éthanol, ladite inductibilité par l'éthanol étant médiée par le produit du gène alcR;
(ii) des conditions dans lesquelles il se produit une expression régulée du polypeptide, et
(iii) la récupération dudit polypeptide.

2. Procédé suivant la revendication 1, dans lequel le promoteur peut être réprimé par le glucose.

3. Procédé suivant la revendication 2, comprenant le culture du champignon filamenteux en présence de glucose durant la phase de croissance de la cellule et l'addition d'un inducteur en l'absence de glucose pour accroître l'activité du promoteur.

4. Procédé selon l'une quelconque des revendications précédentes, dans lequel le promoteur est dérivé d'un gène de l'alcool déshydrogénase ou d'un gène de l'aldéhyde déshydrogénase d'un champignon filamenteux.

5. Procédé selon l'une quelconque des revendications précédentes, dans lequel la construction d'ADN contenant le promoteur régulable est présente en plusieurs copies.

6. Procédé suivant la revendication 5, dans lequel la souche de champignon filamenteux exprime des niveaux d'alcR supérieurs à la normale, augmentant ainsi le niveau d'expression des copies multiples de la construction d'ADN contenant le promoteur régulable.

7. Procédé selon l'une quelconque des revendications 1 à 6, dans lequel le promoteur régulable est dérivé d'un gène d'Aspergillus.

8. Procédé suivant la revendication 7, dans lequel le promoteur régulable est dérivé d'un gène d'Aspergillus nidulans.

9. Procédé suivant la revendication 7, dans lequel le promoteur régulable est dérivé d'un gène d'Aspergillus niger.

10. Procédé selon l'une quelconque des revendications précédentes, dans lequel le champignon filamenteux est un Aspergillus.

11. Procédé suivant la revendication 10, dans lequel ledit Aspergillus est A. nidulans.

12. Procédé suivant la revendication 10, dans lequel ledit Aspergillus est A. niger.

13. Champignon filamenteux comme défini dans l'une quelconque des revendications 1, 2 ou 4 à 12.

14. Construction d'ADN recombinant, comprenant un promoteur régulable d'un gène de champignon filamenteux lié de façon opérante à un fragment d'ADN codant pour le polypeptide, dans laquelle :
(a) ledit fragment d'ADN est étranger au promoteur, et
(b) ledit promoteur régulable peut être induit par l'éthanol, ladite inductibilité par l'éthanol étant médiée par le produit du gène alcR.

15. Construction d'ADN recombinant suivant la revendication 14, qui est comme défini plus avant dans l'une quelconque des revendications 2 ou 4 à 12.
